# EUROPEAN PATENT APPLICATION

(11) **EP 1 811 297 A1**
(43) Date of publication of application: **25.07.2007**
(21) Application number: 06001053.5
(22) Date of filing: 18.01.2006
(51) Int. Cl.: G01N 33/50, G01N 33/68

(54) **Test systems for the analysis of polypeptides and cells adhering to silicones**

(71) Applicant: IDC Immunological Diagnostic and Consulting KG, 6020 Innsbruck (AT)
(72) Inventor: Wick, Georg, 6080 Igls/Innsbruck (AT); Wick, Nikolaus, 6080 Igls/Innsbruck (AT); Backovic, Aleksandar, 6020 Innsbruck (AT)
(74) Representative: Krauss, Jan

(57) **Abstract**

The present invention relates in general to the field of diagnostics, namely to test systems for polypeptides in respect to silicone. More precisely, the present invention, in one embodiment thereof, concerns test systems for identifying and analysing polypeptides adhering to silicone. Furthermore, the present invention relates to newly identified polypeptides adhering to silicone and to respective uses of such proteins, e.g., in test systems for detecting a response reaction of a mammal to a silicone.

## Description

The present invention relates in general to the field of diagnostics, namely to test systems for polypeptides in respect to silicone. More precisely, the present invention, in one embodiment thereof, concerns test systems for identifying and analyzing polypeptides adhering to silicone. Furthermore, the present invention relates to newly identified polypeptides adhering to silicone and to respective uses of such proteins, e.g., in test systems for detecting a response reaction of a mammal to a silicone.

### BACKGROUND OF THE INVENTION

Dimethylsiloxane polymer, also known as 'silicone', is the most widely utilized inorganic biomaterial in medical practice, because of its assumed biocompatibility. It is used in a large variety of applications, such as medical tubing systems, syringe lubricants, coating of active medical devices like bypass apparatus and cochlear implants, and for the production of passive implants ranging from joint replacements and eye lenses to ventriculo-peritoneal shunts, and mammary implants.

However, particularly after long-term implantation, various local and systemic side effects have been reported as a response reaction to silicone. These phenomena have been observed, for example, in women with silicone mammary implants (SMIs) [Smalley, D. L., Levine, J. J., Shanklin, D. R., Hall, M. F., and Stevens, M. V. Lymphocyte response to silica among offspring of silicone breast implant recipients. Immunobiology 196, 567-574 (1996)], in most cases of failures of sterile ventriculo-peritoneal shunts [Goldblum, R. M., Pelley, R. P., O'Donell, A. A., Pyron, D., and Heggers, J. P. Antibodies to silicone elastomers and reactions to ventriculoperitoneal shunts. Lancet 340, 510-513 (1992)], and also in patients carrying various silicone coated medical devices [Kinnari, T. J., Salonen, E. M., and Jero, J. Durability of the binding inhibition of albumin coating on tympanostomy tubes. International Journal of Pediatric Otorhinolaryngology 67, 157-164 (2003)].

The so-called local reaction of the organism against the silicone is characterized by the formation of a fibrotic connective tissue capsule that can vary in extent [Siggelkow, W., Faridi, A., Spiritus, K., Klinge, U., Rath, W., and Klosterhalfen, B. Histological analysis of silicone breast implant capsules and correlation with capsular contracture. Biomaterials 24, 1101-1109 (2003)]. Over the course of years, the fibrosis progresses causing functional impairment of the implant. Local side effects are often followed by systemic problems, and there have been reports on patients with long term silicone implants that have been diagnosed with fibromyalgia [Vermeulen, R. C. and Scholte, H. R. Rupture of silicone gel breast implants and symptoms of pain and fatigue. The Journal of Rheumatology 30, 2263-2267 (2003); Shanklin, D. R., Stevens, M. V., Hall, M. F., and Smalley, D. L. Environmental immunogens and T-cell-mediated responses in fibromyalgia: evidence for immune dysregulation and determinants of granuloma formation. Experimental and Molecular Pathology 69, 102-118 (2000)], rheumatic and other types of connective tissue disease (CTD) [Bridges, A. J., Conley, C., Wang, G., Bums, D. E., and Vasey, F. B. A clinical and immunologic evaluation of women with silicone breast implants and symptoms of rheumatic disease. Ann Intern Med 118, 929-936 (1993)], and even with a scleroderma-like fibrotic syndrome [Varga, J., Schumacher, H. R., and Jimenez, S. A. Systemic sclerosis after augmentation mammoplasty with silicone implants. Cancer Research 111, 377-383 (1989); Appleton, B. E. and Lee, P. The development of systemic sclerosis (scleroderma) following augmentation mammoplasty. J Rheumatol 20, 1052-1054 (1998)].

Although it is generally believed that the systemic side effects reflect changes on the local level, such a crosstalk has never been defined. Even more important, prospective cohort studies, both in Europe and the United States, could neither confirm nor deny the effect of silicone implants as a risk factor for the development of CTD or other autoimmune diseases [Janowsky, E. C., Kupper, L. L., and Hulka, B. S. Meta-analyses of the relation between silicone breast implants and the risk of connective-tissue diseases. New England Journal of Medicine 342, 781-790 (2000); Sanchez-Guerrero, J., Colditz, G. A., Karlson, E. W., Hunter, D. J., Speizer, F. E., and Liang, M. H. Silicone Breast Implants and the Risk of Connective-Tissue Diseases and Symptoms. N Engl J Med 332, 1666-1670 (1995)].

Nevertheless, it has been shown that connective tissue disease-like symptoms in carriers of SMIs disappeared after the removal of the implant [Zazgornik, J., Piza, H., Kaiser, W., Bettelheim, P., Steiner, G., Smolen, J., Biesenbach, G., and Maschek, W. Autoimmune reactions in patients with silicone breast implants. Wien. Klin. Wochenschr. 108, 781-787 (1996)]. Furthermore, it could be shown that there is a contribution of immunological mechanism to the development of peri-SMI capsule formation [Wolfram, D., Rainer, C., Niederegger, H., Piza, H., and Wick, G. Cellular and molecular composition of fibrous capsules formed around silicone breast implants with special focus on local immune reactions. J Autoimmun. 23, 81-91 (2004)]. Thus, it was demonstrated that within these capsules delayed type hypersensitivity reactions take place.

Most experts agree that the reaction to silicone is a type of low intensity perpetuated foreign body reaction, but the mechanisms behind those processes are not exactly known. Even though activation of immune response to silicone has been demonstrated, it is not clear, whether (i) silicone itself has antigenic properties, (ii) acts as a hapten or carrier, (iii) represents an immunological adjuvant, (iv) is a surface that promotes exposure of cryptic antigens and 'altered self, or (v) induces a stress response in the surrounding tissue.

Even though it has been identified as a key factor in an immune reaction to silicone, the composition of the proteinaceous film on the surface of silicone is poorly understood. It is acquired on the implanted silicone surface almost instantaneously after the implantation [Elwing, H. Protein absorption and ellipsometry in biomaterial research. *Biomaterials* **19*,*** 397-406 (1998)]. Proteins forming the film are recognized by phagocytes and fibroblasts that are the first cells interacting with the implant.

It is important to emphasize that in biological systems, flow conditions, protein conformational changes, competitive adsorption of proteins, and early effects in blood clotting and complement activation strongly influence the formation of the protein film on the artificial surfaces ('Vroman effect') [Vroman, L., Adams, A.L., Fischer, G.C., and Munoz, P.C. Interaction of High Molecular-Weight Kininogen, Factor-Xii, and Fibrinogen in Plasma at Interfaces. Blood 55, 156-159 (1980)]. Because of the complexity of the film, it has so far been only shown that the γ190-202 cryptic immunogenic epitope of the fibrinogen D domain is able to activate macrophages [through MAC-1 (CD11b/CD18) on their surface] [Hu, W.J., Eaton, J.W., Ugarova, T.P., and Tang, L. Molecular basis of biomaterial-mediated foreign body reactions. Blood 98, 1231-1238 (2001)]. This epitope of fibrinogen is exposed only upon adhesion to a silicone surface and plays a crucial role in local side effects of SMIs. As a response, activated macrophages produce TNF-α, leading to the recruitment of lymphocytes and inducing transdifferentiation, proliferation and migration of fibroblasts to the surrounding tissue. Fibrosis occurs as the response of the surrounding tissue to the chronic inflammatory reaction to silicone, constantly perpetuated by lymphocyte, fibroblasts and macrophages interacting with proteins adhered to silicone surface.

More evidence for the immune hypothesis of the fibrotic capsule formation can be found in the composition of the peri-implant connective tissue capsule. Capsule formation has been investigated on an immunohistological level in numerous studies with similar findings [Wick, G., Wagner, R., Klima, G., and Wilflingseder, P. in Cellular, Molecular and Genetic Approaches to Immunodiagnosis and Immunotherapy, eds. Kano, K., Mori, S., Sugisaki, T., and Torisu, M. 231-241 (University of Tokyo Press, Tokyo; 1987) and Gabbiani, G., Hirschel, B.J., Ryan, G.B., Statkov, P. R., and Majno, G. Granulation tissue as a contractile organ. A study of structure and function. The Journal of Experimental Medicine 135, 719-734 (1972)].

It has been shown that a synovial-like multilayer metaplasia composed of cells with macrophage and fibroblastic phenotype is formed towards the implant. Interestingly, these cells also express abundant amount of stress proteins, like Heat Shock Protein 60 (HSP60), on the surface of SMIs [Wolfram, D., Rainer, C., Niederegger, H., Piza, H., and Wick, G. Cellular and molecular composition of fibrous capsules formed around silicone breast implants with special focus on local immune reactions. J Autoimmun. 23, 81-91 (2004)]. The chronic proliferative inflammation pattern of fibroblasts is resulting in dense collagenous fibrosis after an implant duration of more than two years [Siggelkow, W., Faridi, A., Klinge, U., Rath, W., and Klosterhalfen, B. Ki67, HSP70 and TUNEL for the specification of testing of silicone breast implants in vivo. J Mater. Sci. Mater. Med 15, 1355-1360 (2004)]. Additional stimulation of local fibrotic processes through secretion of Connective Tissue Growth Factor (CTGF) is mediated by γδ-T-cells in the capsule [Workalemahu, G., Foerster, M., Kroegel, C., and Braun, R. K. Human gamma delta-T lymphocytes express and synthesize connective tissue growth factor: effect of IL-15 and TGF-beta 1 and comparison with alpha beta-T lymphocytes. The Journal of Biological Chemistry 170, 153-157 (2003)], which was also found in the peri-implant connective tissue capsule. In addition, macrophages are stimulated via a positive feedback loop by TNF-α produced by γδ-T-cells.

US 4,048,298 discloses a solid double-antibody radioimmunoassay procedure. The patent concerns a radioimmunoassay (RIA) procedure for assaying body fluid content of an antigenic substance which may either be an antigen itself or a hapten capable of being converted, such as by means of reaction with a protein, to an antigenic material. This invention relates to a novel and improved modification of a double-antibody RIA technique in which there is a first antibody that is specific to the antigenic substance suspected to be present in a body fluid from which the assay is intended. The second antibody, however, is not specific to the antigenic substance or analyte, but is an antibody against the first antibody.

WO 02/41000 discloses an immunoassay method for membrane-bound matrix metalloprotease. By using a simple procedure and a reagent, MT1-MMP can be quickly quantified or the enzymatic activity of MT1-MMP can be measured at a high sensitivity and a high accuracy. Namely, use of a method of immunologically quantifying MT1-MMP by using an anti-MT1-MMP antibody and a reagent to be used therewith; a method of immunologically quantifying a member selected from the group consisting of (i) MT1-MMP having been released and/or solubilized from cell membrane with the use of a surfactant and/or a reducing agent and (ii) autonomously solubilized MT1-MMP and a reagent to be used therewith; and solid-phase MT1-MMP makes it possible to quantify MT1-MMP or measure the enzymatic activity of MT1-MMP. Moreover, an MT1-MMP expression promoter or inhibitor and an MT1-MMP activity promoter or inhibitor can be screened thereby. Thus, research and development of useful drugs can be facilitated. These reagents are also useful in examining cancer and cancer metastasis.

Silicone breast implants have a controversial history and are no longer approved for use by the Food and Drug Administration. They consisted of a flexible silicone rubber outer envelope that was filled with a silicone gel. Anecdotal reports of complications related to silicone breast implants are numerous including associations with connective tissue disorders such as scleroderma and rheumatoid syndromes.

Indisputable complications of all types of breast implants include surgically related complications such as bleeding and infection. Later in the life of an implant, the predominant complications include capsular contracture and implant rupture. However, there are analogous complications against the outer envelope of an intact silicone implant.

The cause of these complications remains elusive although some interesting things have been demonstrated. For instance, the gel inside the silicone implant is in a polymerized form. Unpolymerized silicone can bleed through an intact silicone envelope into the surrounding tissue. It is theorized that this unpolymerized silicone stimulates the fibrotic reaction responsible for capsular contraction. Silicone bleeding through an intact envelope, as well as implant rupture, is associated with granuloma formation. Nevertheless, also polymerised silicone that covers the surface of an intact implant, i.e. the "true" surface, has a potential for a granulomatous or fibrotic capsule formation, i.e. even without bleeding of the silicone.

Currently, the interaction between proteins and silicone is only little understood. Thus, there is a need to provide methods and devices in order to further explore the role of silicone-adhering proteins and the proteinaceous film, and in particular the interaction of cells and extracellular matrix (ECM) proteins with silicone, and to identify novel proteins that are able to adhere to silicone surfaces. A better understanding of the mechanisms underlying the reaction of a subject to silicone is essential, for example, if one aims at identifying individuals prone to develop local or systemic side effects as described above and to base appropriate methods for monitoring, prevention and therapy on these results. In addition, a better understanding also plays a crucial role for providing silicone having a higher biocompatibility.

### SUMMARY OF THE INVENTION

In a first aspect thereof, the present invention provides a method for identifying polypeptides adhering to silicone surface, comprising the following steps of: a) providing a sample from a mammal, wherein said sample is suspected to contain silicone-adhering polypeptides; b) optionally, providing said sample with a silicone, in particular a medical silicone; c) eluting of polypeptides that are adhered to said silicone in said sample; and d) identifying said eluted polypeptides.

In a further aspect thereof, the present invention relates to a reagent kit for identifying polypeptides adhering to a silicone surface, comprising materials, buffers and auxiliary agents and substances for performing a method according to the present invention.

In a further aspect thereof, the present invention provides silicone-adhering polypeptides identified through a method or a kit according to the present invention.

In one preferred embodiment, the present invention relates to the use of a polypeptide identified according to the present invention for detecting a response reaction of a mammal to a silicone, in particular a medical silicone.

In another preferred embodiment, the present invention relates to a pharmaceutical composition comprising at least one silicone-adhering polypeptide identified according to the present invention, and a pharmaceutical carrier.

In another preferred embodiment, the present invention provides a polypeptide comprising an amino acid sequence selected from the group consisting of SEQ ID NO. 1, SEQ ID NO. 2, SEQ ID NO. 3, and SEQ ID NO. 4 or a functional variant thereof.

In yet another preferred embodiment, the present invention relates to a nucleic acid comprising a nucleotide sequence encoding for a polypeptide according to the present invention, in particular for a polypeptide having the amino acid sequence set forth in any one of SEQ ID NO. 1, SEQ ID NO. 2, SEQ ID NO. 3, or SEQ ID NO. 4, or a functional variant thereof, or a complementary nucleotide sequence of said nucleic acid.

In yet another preferred embodiment, the present invention relates to a nucleic acid that hybridizes under stringent conditions with the nucleic acid according to the present invention. In another preferred embodiment the present invention provides a recombinant vector, comprising the nucleic acid according to the present invention. In a preferred further embodiment, the present invention concerns a host cell comprising a nucleic acid or vector or an expression vector according to the present invention. In a further preferred embodiment, the present invention relates to an antibody that immunologically recognizes a polypeptide according to the present invention. In another preferred embodiment, the present invention provides a polypeptide, a nucleic acid or vector or an expression vector according to the present invention for use in medicine.

In another preferred embodiment the present invention relates to an antibody that immunologically recognizes a polypeptide comprising an amino acid sequence selected from the group consisting of SEQ ID NO. 1, SEQ ID NO. 2, SEQ ID NO. 3, and SEQ ID NO. 4 or a functional variant thereof.

In one further preferred embodiment, the present invention concerns a pharmaceutical composition, comprising an effective dose of a polypeptide according to the present invention, a nucleic acid or vector according to the present invention, an expression vector according to the present invention, a host cell according to the present invention, or an antibody according to the present invention, and a pharmaceutically acceptable carrier.

In a further preferred embodiment, the present invention relates to the use of a polypeptide according to the present invention, a nucleic acid or vector according to the present invention, an expression vector according to the present invention, a host cell according to the present invention, or an antibody according to the present invention for detecting a response reaction of a mammal to a silicone, in particular to a medical silicone.

In yet another preferred embodiment, the present invention concerns the use of a polypeptide for detecting a response reaction of a mammal to a silicone, wherein said polypeptide is selected from the group consisting of Myeloid related protein 8, Myeloid related protein 14, HT018, and PRO2619 or a functional variant thereof.

In another preferred embodiment, the present invention relates to a method of diagnosing a response reaction of a mammal to a silicone, comprising the steps of: a) providing a sample from a mammal to be diagnosed; b) detecting the presence or a change in expression of at least one silicone-adhering polypeptide or a functional variant thereof in said sample; and c) concluding from said presence or change in expression of said at least one silicone-adhering polypeptide or functional variant thereof on a response reaction of said mammal to said silicone.

In a further preferred embodiment, the present invention relates to a diagnostic kit for detecting a response reaction in a mammal against silicone, in particular medical silicone, comprising materials, buffers, auxiliary agents, and substances for performing a method according to the present invention.

In another preferred embodiment, the present invention relates to a method for monitoring a response reaction of a mammal to a silicone, in particular medical silicone, comprising detecting the presence or a change in expression of a silicone-adhering polypeptide using a method or kit according to the present invention.

In another preferred aspect thereof, the present invention provides a method for screening substances that interfere with at least one silicone-adhering polypeptide, comprising the steps of a) providing a substance, wherein said substance is suspected to interfere with the adhering properties of at least one silicone-adhering polypeptide; b) providing a silicone, in particular a medical silicone; c) providing a silicone-adhering polypeptide; and, d) analyzing the interfering properties of said substance through analyzing the adhesion of said silicone-adhering polypeptide to said silicone in the presence and absence of said substance.

In another preferred aspect thereof, the present invention relates to a method for detecting cell adhesion to silicone, comprising a) providing a sample of serum or wound bed fluid of a mammal; b) providing of silicone; and c) analyzing the amount or number of cells from said serum or wound bed fluid that are adhered to said silicone.

In another preferred embodiment, the present invention provides a method of treatment of a mammal suffering from a response reaction to a silicone, comprising administering to said mammal an effective amount of a substance interfering with the adhering properties of a silicone-adhering polypeptide as identified through a method according to the present invention.

In another preferred embodiment the present invention provides a method for monitoring the treatment of a mammal suffering from a response reaction to a silicone, comprising: a) administering to said mammal an effective amount of a substance interfering with the adhering properties of a silicone-adhering polypeptide as identified according to the present invention b) obtaining a sample from said mammal; and c) detecting the presence or a change in expression of at least one silicone-adhering polypeptide or a functional variant thereof.

### DESCRIPTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purposes of the present invention, all references as cited herein are incorporated by reference in their entireties.

As outlined above there is a need in the prior art to identify polypeptides which are involved for a response reaction of a mammal to a silicone. These polypeptides presumably play a role as key activators of the immune response, which is in turn responsible for the fibrosis leading to implant failure. Thus, a detailed analysis of their composition and the dynamics of their deposition is necessarily. Furthermore, these identified polypeptides might serve as both diagnostic as well as potential therapeutic target molecules with respect to the response reaction to silicone.

The term "response reaction" as used herein, shall mean the physiological reaction of a body or a cell to a silicone, such as a medical silicone. Such reaction can manifest itself as an immunological response involving the immune system of the body, and can be present as a local (i.e. at or closely around the site of the contact with the silicone) or systemic (i.e. generally found in the body, such as, for example, in the blood, serum or other fluids) side effect, for example as a response to an implanted silicone, such as to implanted SMIs [Smalley, D. L., Levine, J. J., Shanklin, D. R., Hall, M. F., and Stevens, M. V. Lymphocyte response to silica among offspring of silicone breast implant recipients. Immunobiology 196, 567-574 (1996)]. The local and systemic side effects presumably result in the formation of a fibrotic connective tissue capsule (usually with a foreign-body granuloma formation), fibromyalgia, connective tissue disease (e.g. inflammation), and scleroderma-like fibrotic syndrome. Of note the fibrotic process occurring in response to a silicone implant are a model for other, non-implant associated fibrotic diseases, of which four different types are known: (i) post-necrotic diseases, e.g. hepatic cirrhosis, (ii) inflammatory diseases, e.g. rheumatoid arthritis, (iii) or spontaneous forms, e.g. Dypuytren's contracture and (iv) foreign-body reactions in general (e.g. reactions against implants).

The term "hypersensitivity" as used herein, refers to a particular type of (an exaggerated) response reaction of a mammal to silicone, e.g. an adverse reaction of an individual in response to a particular silicone, which, in one embodiment, can only be found in this individual, whereas other individuals might not show any respective response (that is, an individual response to a certain silicone).

In a preferred aspect thereof, the present invention provides a method for identifying polypeptides adhering to silicone, comprising the following steps of: a) providing a sample from a mammal, wherein said sample is suspected to contain silicone-adhering polypeptides; b) optionally, providing said sample with a silicone; c) eluting of polypeptides that are adhered to said silicone in said sample; and d) identifying said eluted polypeptides. Preferably, the polypeptides are first separated using gel electrophoresis, more preferably two-dimensional gel electrophoresis. Preferably, this step is then followed by a staining procedure known to a person skilled in the art, preferably silver staining. The thus stained polypeptides will be detectable as a polypeptide "spot pattern". This pattern is matched and compared with e.g. a control pattern or with a database, e.g. with the ExPasy database of 2D gels, using a software. Preferably, a polypeptide spot of interest is excised, more preferably the polypeptide spot of interest is excised manually. Then, the excised polypeptide spot is preferably digested to obtain fragments that are suitable to be analyzed by mass spectrometry, e.g. peptide mass fingerprint (PMF) using MALDI-TOF. Preferably, the samples are further analyzed using LIFT-TOF/ TOF MS/MS. More preferably, the results are then confirmed using Western blot analysis as known by a person skilled in the art.

The terms "adhere" or "adhesion" as used herein, refers to the firmly sticking of a compound, e.g. a polypeptide to a surface, e.g., the surface of a silicone. This sticking can be performed by both covalent and non-covalent binding between the polypeptide and the silicone.

The term "polypeptide", in accordance with the present invention, shall mean a peptide or a protein which encompasses amino acid chains of a given length of at least 10, preferably, 20-50, more preferred 30-100 amino acids, wherein the amino acid residues are linked by covalent peptide bonds. However, peptidomimetics of such polypeptides wherein amino acid(s) and/or peptide bond(s) have been replaced by functional analogs are also encompassed by the invention. A most preferred polypeptide is a part of the peptide that is functionally responsible or involved for the adhesion of the polypeptide to silicone.

The term "silicone" as used herein, shall encompass all dimethylsiloxane polymers that can be used in a physiological environment, such as the human body. Usually, such silicones show a biocompatibility (i.e. do induce no or only slight immune responses of the body) and preferably have a high physiological acceptance. Such silicones are also known as "medical silicones". Such medical silicones can be used for medical implants, such as, for example, silicones mammary implants or as coatings for medical devices e.g. bypass implants. Such medical silicones suitable to be used according to the present invention are known by the person skilled in the art. Preferred examples of such medical silicones are the silicone types MED1511, MED4860, and MED4211 provided by the company Nusil (Waldbroon, Germany).

In a preferred embodiment of the method for identifying polypeptides adhering to silicone according to an aspect of the present invention, the sample is selected from a proteinaceous film covering a silicone, urine, pleural- or peritoneal fluid, cerebrospinal fluid, wound bed fluid, or serum. Preferably said sample is selected from a proteinaceous film covering a silicone, serum or wound bed fluid. Preferably said wound bed fluid is preferably obtained with drainage.

In a preferred embodiment of the method for identifying polypeptides adhering to silicone according to the present invention the sample is obtained from a mammal showing a response reaction to silicone.

In another preferred embodiment of the method for identifying polypeptides adhering to silicone according to the present invention the sample is obtained from a mammal showing a response reaction selected from the group consisting of a fibrotic disease of one of the following types: (i) post-necrotic diseases, e.g. hepatocirrhosis, (ii) inflammatory diseases, e.g. rheumatoid arthritis, (iii) spontaneous fibrotic diseases, e.g. Dupuytren's contracture, (iv) and foreign-body reaction in general. Such diseases can result in the formation of a fibrotic connective tissue capsule, fibromyalgia, connective tissue diseases, and scleroderma-like fibrotic syndrome.

In a further preferred embodiment of the method for identifying polypeptides adhering to silicone according to the present invention the fibrotic connective tissue capsule is formed through an immunological response.

In a preferred embodiment of the method for identifying polypeptides adhering to silicone according to the present invention, the wound bed fluid or serum is provided together with an additional silicone, preferably with a medical silicone.

In a preferred embodiment of the method for identifying polypeptides adhering to silicone according to the present invention the silicone is provided unpolymerized. The term "unpolymerized" as used in accordance with the present invention relates to the state of a silicone, e.g. a medical silicone, which is essentially not polymerized. Unpolymerized silicone usually occurs as a by-product of the polymerization of silicone implants (in particular breast implants) and is believed to cause many of the problems associated with such implants.

In another preferred embodiment of the method for identifying polypeptides adhering to silicone according to the present invention the silicone can be any medical silicone known by the person skilled in the art. Preferably the silicone is selected from the group consisting of the silicone types MED1511, MED4860, and MED4211 provided by the company Nusil (Waldbroon, Germany).

In another preferred embodiment of the method for identifying polypeptides adhering to silicone according to the present invention step d) comprises any method known by the person skilled in the art. Preferably, step d) comprises methods selected from one or two-dimensional electrophoresis, Western blot analysis, protein assays suitable to measure the amount and/or presence of said proteins, mass spectrometry, a method suitable for analyzing modifications, e.g. silver staining, advanced glycation end products (AGE) formation, oxidation, antibody arrays, ligand arrays/assays, enzyme immunoassay (EIA, e.g. ELISA), radioimmunoassay (RIA), fluorescence immunoassay (FIA), and Luminex assay.

In a preferred embodiment of the method for identifying polypeptides adhering to a silicone according to the present invention, the method further comprises in step e) further identifying of polypeptides that are suitable as marker polypeptides for detecting a response reaction in a mammal to silicone. Preferably, proteins eluted from proteinaceous film covering explanted silicone are compared to silicone-adhering proteins obtained from wound bed fluid or serum. More preferably, the proteins eluted from the two systems are compared by one and/or two-dimensional gel electrophoresis. Preferably, proteins from the two systems are labeled with different fluorochromes that can be excited at specific wavelengths, leading to independent images from a single gel. Nevertheless, also a radioactive detection can be carried out. Respective labels are well known to the person of skill. Preferably, the protein spot patterns are analyzed with any software suitable to analyze such patterns known by a person skilled in the art. Preferably, the protein spot patterns are analyzed with the 'Decyder' software (Amersham Biosciences).

In another preferred embodiment of the method for identifying polypeptides adhering to silicone according to the present invention the response reaction is a hypersensitivity reaction in a mammal to silicone.

In a preferred embodiment of the method for identifying polypeptides adhering to silicone according to the present invention, step e) comprises: e') comparing polypeptide patterns derived from the separation of said proteins eluted from explanted silicone with polypeptide patterns derived from the separation of silicone-adhering polypeptides eluted from wound bed fluid or serum; and e") selecting and analyzing a spot of interest. Preferably, the amount of proteins deposited are considered different, if the spot volume ratio between samples exceeded two standard deviations of total spot distribution, most preferably, a spot is defined as present only in a system, if the volume ratio exceeded a threshold of fivefold.

In another preferred embodiment of the method for identifying polypeptides adhering to silicone according to the present invention in step a) the polypeptides are furthermore labeled with different fluorochromes or are radioactive labeled. Preferably the fluorochromes are excitable at specific wavelengths. Any fluorochrome known by the person skilled in the art can be used. Preferably the fluorochromes are selected from the group consisting of 7-aminoactinomycine d (7-AAD), acridine orange, alexa dyes, amino methylcoumarine-acetate (AMCA), Cy-dyes, e.g. Cy2, Cy3, Cy5, Cy7, 4',6-Diamidino-2-phenylindole dihydrochloride (DAPI), fluorescein isothiocyanate (FITC), Hoechst-dyes, e.g., Hoechst 33342, Hoechst 33258, "blue" fluorescence, 5,5',6,6'Tetrachloro-1,1',3,3'-tetraethylbenzimidazolylcarbocyanine jodide (JC-1), peridinin-chlorophyll-protein (PerCP), propidium jodide, rhodamine Red-X, phycoerythrin (R-PE), Aminomethylcoumarin acetate sulfonyl chloride rhodamine -derivate (Texas-Red), and Tetramethyl rhodamine isothiocyanate (TRITC).

In a preferred embodiment of the method for identifying polypeptides adhering to silicone according to the present invention in step e") polypeptide spot patterns are analyzed by searching for all spots presented in the polypeptide patterns from polypeptides eluted from silicone and presented in the polypeptide patterns of polypeptides of separated silicone-adhering polypeptides eluted from wound bed fluid or serum.

In a particular preferred embodiment of the method according to the present invention, the mammal is human.

In a further aspect thereof, the present invention provides a reagent kit for identifying polypeptides adhering to a silicone, comprising materials, buffers and auxiliary agents and substances for performing the method for identifying polypeptides adhering to silicone surface according to the present invention.

In a further aspect thereof, the present invention relates to a silicone-adhering polypeptide identified through a method or a kit for identifying polypeptides adhering to silicone surface according to the present invention.

In a further aspect thereof, the present invention relates to the use of a polypeptide identified by a method for identifying polypeptides adhering to a silicone according to the present invention for detecting a response reaction of a mammal to a silicone.

In a further aspect thereof, the present invention relates to a pharmaceutical composition, comprising at least one polypeptide identified by a method for identifying polypeptides adhering to a silicone according to the present invention and a pharmaceutical carrier.

In a further aspect thereof, the present invention relates to a polypeptide comprising an amino acid sequence selected from the group consisting of SEQ ID NO. 1, SEQ ID NO. 2, SEQ ID NO. 3, and SEQ ID NO. 4 or a functional variant thereof.

The term "functional variants" in the sense of the present invention means polypeptides that are functionally related to the polypeptides of the present invention, i.e. share the common structural or functional feature of the polypeptides, to adhere to silicone. Examples of functional variants are orthologs, i.e. the corresponding polypeptide derived from another organism as human, preferably from non-human mammals such as, for example, monkeys, pigs, mice, and rats. Other examples of functional variants are polypeptides that are encoded by different alleles of the gene, in different individuals or in different organ of an organism. Furthermore, also polypeptides shall be encompassed by said term that have a sequence homology, in particular a sequence identity, of about 70%, preferably about 80%, in particular about 90%, more preferred about 95% to the polypeptide with the amino acid sequence according to the polypeptides of the present invention and/or DNA sequences that are derived from these polypeptide sequences. Included are additions, inversions, substitutions, deletions, insertions or chemical/physical modifications and/or exchanges or parts of the polypeptide in the range of about 1-60, preferably of about 1-30, and most preferred about 1-15 amino acids. Thus, functional variants preferably can encompass domain(s) of the protein that are responsible for the adhesion of said polypeptide to a silicone.

In a preferred embodiment of the polypeptide according to the present invention the polypeptide or the functional variant thereof adheres to a silicone.

In another preferred embodiment of the polypeptide according to the present invention, the polypeptide is a fusion protein.

The term "fusion protein" as used herein, denotes any polypeptide consisting or comprising of at least two polypeptides not naturally forming such a polypeptide. On the DNA level, the two or more coding sequences are fused in frame. The parts of the fusion protein can be connected and/or spaced apart by linkers (usually peptide linkers or chemical linkers) that are known to the person of skill.

In a another aspect thereof, the present invention relates a nucleic acid comprising a nucleotide sequence encoding for a polypeptide according to the present invention, in particular for a polypeptide having the amino acid sequence set forth in any one of SEQ ID NO. 1, SEQ ID NO. 2, SEQ ID NO. 3, or SEQ ID NO. 4, or functional variants thereof, or a complementary nucleotide sequence of said nucleic acid.

The term "functional variants" designates all DNA-sequences that are complementary to a DNA-sequence that hybridize under stringent conditions with a derived reference sequence or parts thereof, and exhibit a similar or identical activity compared to the respective peptide of the present invention. Hybridization conditions are known to those skilled in the art and can be found in Current Protocols in Molecular Biology, John Wiley & Sons, N.Y., 6.3.1-6.3.6, 1991. Moderate hybridization conditions are defined as equivalent to hybridization in 2X sodium chloride/sodium citrate (SSC) at 30°C, followed by a wash in 1 X SSC, 0.1% SDS at 50°C. Highly stringent conditions are defined as equivalent to hybridization in 6X sodium chloride/sodium citrate (SSC) at 45°C, followed by a wash in 0.2 X SSC, 0.1 % SDS at 65°C.

In a preferred embodiment of the nucleic acid according to the present invention, the nucleic acid is DNA, cDNA, PNA, CNA, RNA or combinations thereof. Furthermore, the nucleic acid molecules of the present invention can be genomic DNA, synthetic DNA, or combinations thereof, and can be double-stranded or single-stranded, the sense- and/or an antisense strand. Segments of these molecules are also considered within the scope of the invention, and can be produced by, for example, the polymerase chain reaction (PCR) or generated by treatment with one or more restriction endonucleases. A ribonucleic acid (RNA) molecule can be produced by *in vitro* transcription.

The nucleic acid molecules of the present invention can contain naturally occurring sequences, or sequences that differ from those that occur naturally, but, due to the degeneracy of the genetic code, encode the same peptide (for example, the peptides with SEQ ID NOs: 1, 2, 3 or 4). In addition, these nucleic acid molecules are not limited to coding sequences, e.g., they can include some or all of the non-coding sequences that lie upstream or downstream from a coding sequence.

The nucleic acid molecules of the invention can be synthesized *in vitro* (for example, by phosphoramidite-based synthesis) or obtained from a cell, such as the cell of a bacterium or mammal. The nucleic acids can be those of a human but also derived from a non-human primate, mouse, rat, guinea pig, cow, sheep, horse, pig, rabbit, dog, or cat. Combinations or modifications of the nucleotides within these types of nucleic acids are also encompassed.

In addition, the isolated nucleic acid molecules of the invention can encompass segments that are not found as such in the natural state. Thus, the invention encompasses recombinant nucleic acid molecules incorporated into a vector (for example, a plasmid or viral vector) or into the genome of a heterologous cell (or the genome of a homologous cell, at a position other than the natural chromosomal location). Recombinant nucleic acid molecules and uses therefore are discussed further below.

Furthermore, the nucleic acid according to the present invention can comprise markers or labels, such as affinity tags, antigen tags, fluorescent groups, quenchers, radioactive groups, dyes, heavy atoms (such as nitrogen), enzymatically detectable groups, enzymatically active groups, etc. All these markers or labels and respective technologies for attaching these are well known to the person of skill.

In another aspect thereof, the present invention provides a nucleic acid that hybridizes under stringent conditions with the nucleic acid according to the present invention.

In another aspect thereof, the present invention relates to a recombinant vector comprising a nucleic acid according to the present invention.

Therefore, the present invention relates in another embodiment to a vector comprising the nucleic acid molecule of this invention. Such a vector is capable of being introduced or of introducing the nucleic acid into a cell. It is preferred that the polypeptides encoded by the introduced nucleic acids are expressed within the cell upon introduction of the vector.

In a preferred embodiment of the recombinant vector according to the present invention, the recombinant vector is a recombinant expression vector, including both overexpression and repression vector.

In a preferred embodiment the vector of the present invention comprises plasmids, phagemids, phages, cosmids, artificial mammalian chromosomes, knock-out or knock-in constructs, viruses, in particular adenoviruses, vaccinia viruses, attenuated vaccinia viruses, canary pox viruses, lentivirus (Chang, L.J. and Gay, E.E. (20001) Curr. Gene Therap. 1:237-251), herpes viruses, in particular Herpes simplex virus (HSV-1, Carlezon, W.A. et al. (2000) Crit. Rev. Neurobiol.), baculovirus, retrovirus, adeno-associated-virus (AAV, Carter, P.J. and Samulski, R.J. (2000) J. Mol. Med. 6:17-27), rhinovirus, human immune deficiency virus (HIV), filovirus and engineered versions thereof (see, for example, Cobinger G. P. et al (2001) Nat. Biotechnol. 19:225-30), virosomes, "naked" DNA liposomes, and nucleic acid coated particles, in particular gold spheres. Particularly preferred are viral vectors like adenoviral vectors or retroviral vectors (Lindemann et al. (1997) Mol. Med. 3:466-76 and Springer et al. (1998) Mol. Cell. 2:549-58). Liposomes are usually small unilamellar or multilamellar vesicles made of cationic, neutral and/or anionic lipids, for example, by ultrasound treatment of liposomal suspensions. The DNA can, for example, be ionically bound to the surface of the liposomes or internally enclosed in the liposome. Suitable lipid mixtures are known in the art and comprise, for example, DOTMA (1,2-dioleyloxpropyl-3-trimethylammoniumbromide), and DPOE (dioleoylphosphatidyl-ethanolamine), which both have been used on a variety of cell lines.

Nucleic acid coated particles are another means for the introduction of nucleic acids into cells using so called "gene guns", which allow the mechanical introduction of particles into the cells. Preferably the particles itself are inert, and therefore, are in a preferred embodiment made out of gold spheres.

In a further aspect of the present invention, the nucleic acid of the present invention is operatively linked to expression control sequences allowing expression in prokaryotic and/or eukaryotic host cells. The transcriptional/translational regulatory elements referred to above include but are not limited to inducible and non-inducible, constitutive, cell cycle regulated, metabolically regulated promoters, enhancers, operators, silencers, repressors and other elements that are known to those skilled in the art and that drive or otherwise regulate gene expression. Such regulatory elements include but are not limited to regulatory elements directing constitutive expression like, for example, promoters transcribed by RNA polymerase III like , e.g., promoters for the snRNA U6 or scRNA 7SK gene, the cytomegalovirus hCMV immediate early gene, the early or late promoters of SV40 adenovirus, viral promoter and activator sequences derived from, e.g., NBV, HCV, HSV, HPV, EBV, HTLV, MMTV or HIV; which allow inducible expression like, for example, CUP-1 promoter, the tet-repressor as employed, for example, in the tet-on or tet-off systems, the lac system, the trp system; regulatory elements directing tissue specific expression, regulatory elements directing cell cycle specific expression like, for example, cdc2, cdc25C or cyclin A; or the TAC system, the TRC system, the major operator and promoter regions of phage A, the control regions of fd coat protein, the promoter for 3-phosphoglycerate kinase, the promoters of acid phosphatase, and the promoters of the yeast α- or a-mating factors.

As used herein, "operatively linked" means incorporated into a genetic construct so that expression control sequences effectively control expression of a coding sequence of interest.

Similarly, the nucleic acids of the present invention can form part of a hybrid gene encoding additional peptide sequences, for example, a sequence that functions as a marker or reporter. Examples of marker and reporter genes include β-lactamase, chloramphenicol acetyltransferase (CAT), adenosine deaminase (ADA), aminoglycoside phosphotransferase (neo^{r}, G418^{r}), dihydrofolate reductase (DHFR), hygromycin-B-phosphotransferase (HPH), thymidine kinase (TK), lacZ (encoding β-galactosidase), and xanthine guanine phosphoribosyltransferase (XGPRT). As with many of the standard procedures associated with the practice of the invention, skilled artisans will be aware of additional useful reagents, for example, additional sequences that can serve the function of a marker or reporter. Generally, the hybrid peptide will include a first portion and a second portion; the first portion being a silicon-adhering polypeptide and the second portion being, for example, the reporter described above or an Ig constant region or part of an Ig constant region, e.g., the CH2 and CH3 domains of IgG2a heavy chain. Other hybrids could include an antigenic tag or His tag to facilitate purification and/or detection. Recombinant nucleic acid molecules can also contain a polynucleotide sequence encoding a silicon-adhering polypeptide operatively linked to a heterologous signal sequence. Such signal sequences can direct the protein to different compartments within the cell and are well known to someone of skill in the art. A preferred signal sequence is a sequence that facilitates secretion of the resulting protein.

In order to express cDNAs encoding a polypeptide according to the present invention, one typically subclones a polypeptide according to the present invention cDNA into an expression vector that contains a strong promoter to direct transcription, a transcription/translation terminator, and a ribosome-binding site for translational initiation. Suitable bacterial promoters are well known in the art, e.g., *E. coli, Bacillus sp.,* and *Salmonella,* and kits for such expression systems are commercially available. Similarly eukaryotic expression systems for mammalian cells, yeast, and insect cells are well known in the art and are also commercially available. The eukaryotic expression vector may be, for example an adenoviral vector, an adeno-associated vector, or a retroviral vector.

In addition to the promoter, the expression vector typically contains a transcription unit or expression cassette that contains all the additional elements required for the expression of the silicon-adhering polypeptide-encoding nucleic acid in host cells. A typical expression cassette thus contains a promoter operatively linked to the nucleic acid sequence encoding the silicon-adhering polypeptide and signals required for efficient polyadenylation of the transcript, ribosome binding sites, and translation termination. Additional elements of the cassette may include, for example enhancers. An expression cassette should also contain a transcription termination region downstream of the structural gene to provide for efficient termination. The termination region may be obtained from the same gene as the promoter sequence or may be obtained from different genes.

The particular expression vector used to transport the genetic information into the cell is not particularly critical. Any of the conventional vectors used for expression in eukaryotic or prokaryotic cells may be used. Standard bacterial expression vectors include plasmids such as pBR322 based plasmids, pSKF, pET23D, and fusion expression systems such as GST and LacZ, but there are many more known in the art to the skilled person that can be usefully employed.

Expression vectors containing regulatory elements from eukaryotic viruses are typically used in eukaryotic expression vectors, e.g., SV40 vectors, papilloma virus vectors, and vectors derived from Epstein-Barr virus. Other exemplary eukaryotic vectors include pMSG, pAV009/A.sup.+, pMTO10/A.sup.+, pMAMneo-5, baculovirus pDSVE, pcDNA3.1, pIRES and any other vector allowing expression of proteins under the direction of the SV40 early promoter, SV40 late promoter, metallothionein promoter, murine mammary tumor virus promoter, Rous sarcoma virus promoter, polyhedrin promoter, or other promoters shown effective for expression in eukaryotic cells. Some expression systems have markers that provide gene amplification such as thymidine kinase, hygromycin B phosphotransferase, and dihydrofolate reductase. Alternatively, high yield expression systems not involving gene amplification are also suitable.

The elements that are typically included in expression vectors also include a replicon that functions in *E. coli,* a gene encoding drug resistance to permit selection of bacteria that harbour recombinant plasmids, and unique restriction sites in nonessential regions of the plasmid to allow insertion of eukaryotic sequences. The particular drug resistance gene chosen is not critical, any of the many drug resistance genes known in the art are suitable. The prokaryotic sequences are optionally chosen such that they do not interfere with the replication of the DNA in eukaryotic cells, if necessary.

Standard transfection methods can be used to produce bacterial, mammalian, yeast or insect cell lines that express large quantities of the receptor, which are then purified using standard techniques.

Any of the well known procedures for introducing foreign nucleotide sequences into host cells may be used. These include the use of calcium phosphate transfection, polybrene, protoplast fusion, electroporation, liposomes, microinjection, plasma vectors, viral vectors and any of the other well known methods for introducing cloned genomic DNA, cDNA, synthetic DNA or other foreign genetic material into a host cell. It is only necessary that the particular genetic engineering procedure used be capable of successfully introducing at least one gene into the host cell capable of expressing the silicone-adhering polypeptide according to the present invention.

After the expression vector is introduced into the cells, the transfected cells may be cultured under conditions favoring expression of the silicon-adhering polypeptide, which is recovered from the culture using standard techniques. For example the cells may be burst open either mechanically or by osmotic shock before being subject to precipitation and chromatography steps, the nature and sequence of which will depend on the particular recombinant material to be recovered. Alternatively, the recombinant peptide may be recovered from the culture medium in which the recombinant cells had been cultured.

In another aspect thereof, the present invention concerns a host cell comprising a nucleic acid or vector according, or an expression vector according to the present invention. The host cells that may be used for purposes of the invention include but are not limited to prokaryotic cells such as bacteria (for example, *E. coli* and *B. subtilis),* which can be transformed with, for example, recombinant bacteriophage DNA, plasmid DNA, or cosmid DNA expression vectors containing the polynucleotide molecules of the invention; simple eukaryotic cells like yeast (for example, *Saccharomyces* and *Pichia),* which can be transformed with, for example, recombinant yeast expression vectors containing the nucleic acid molecule of the invention; insect cell systems like, for example, Sf9 of Hi5 cells, which can be infected with, for example, recombinant virus expression vectors (for example, baculovirus) containing the nucleic acid molecules of the invention; Xenopus oocytes, which can be injected with, for example, plasmids; plant cell systems, which can be infected with, for example, recombinant virus expression vectors (for example, cauliflower mosaic virus (CaMV) or tobacco mosaic virus (TMV)) or transformed with recombinant plasmid expression vectors containing a silicon-adhering polypeptide nucleic acid sequence; or mammalian cell systems (for example, COS, CHO, BHK, HEK293, VERO, HeLa, MDCK, Wi38, and NIH 3T3 cells), which can be transformed with recombinant expression constructs containing, for example, promoters derived, for example, from the genome of mammalian cells (for example, the metallothionein promoter) from mammalian viruses (for example, the adenovirus late promoter and the vaccinia virus 7.5K promoter) or from bacterial cells (for example, the tet-repressor binding its employed in the tet-on and tet-off systems). Also useful as host cells are primary or secondary cells obtained directly from a mammal and transfected with a plasmid vector or infected with a viral vector. Depending on the host cell and the respective vector used to introduce the nucleic acid of the invention the nucleic acid can integrate, for example, into the chromosome or the mitochondrial DNA or can be maintained extrachromosomally like, for example, episomally or can be only transiently comprised in the cells.

In another aspect thereof, the present inventions provides an antibody that immunologically recognizes a polypeptide and/or protein according to the present invention. The term "antibody" comprises monoclonal and polyclonal antibodies and binding fragments thereof, in particular Fc-fragments as well as so called "single-chain-antibodies" (Bird R. E. et al (1988) Science 242:423-6), chimeric, humanized, in particular CDR-gafted antibodies, and dia or tetrabodies (Holliger P. et al (1993) Proc. Natl. Acad. Sci. U.S.A. 90:6444-8). Also comprised are immunoglobulin like proteins that are selected through techniques including, for example, phage display to specifically bind to the peptides of the present invention. Preferred antibodies bind to those parts of the peptide responsible for binding to their respective receptor.

In another aspect thereof, the present inventions relates to a polypeptide according to the present invention, a nucleic acid or vector, or an expression vector according to the present invention for use in medicine.

In another aspect thereof, the present inventions concerns to a pharmaceutical composition, comprising an effective dose of a polypeptide according to the present invention, a nucleic acid or vector according to the present invention, or an expression vector according to the present invention, a host cell according to the present invention, or an antibody according to the present invention, and a pharmaceutically acceptable carrier and/or other suitable pharmaceutical auxiliary agents.

The production of pharmaceutical compositions, e.g. in form of medicaments with a content at least one of a polypeptide according to the present invention, a nucleic acid or vector according to the present invention or an expression vector according to the present invention, a host cell according to the present invention or an antibody according to the present invention (in the following simply designated as "active ingredients") and their uses according to the present invention occurs according to standard pharmaceutical technology and methods. For this, the active ingredients, together with pharmaceutical acceptable carriers and/or other suitable pharmaceutical auxiliary agents, are produced into medical forms that suitable for the different indications, and places of administration.

Thereby, pharmaceutical compositions can be produced in a manner that the release rate that is desired, e.g. a quick onset and/or a retard- or depot-effect is achieved. Thereby, the pharmaceutical compositions can be an ointment, gel, patch, emulsion, lotion, foam, creme or mixed-phase or amphiphilic emulsion systems (oil/water-water/oil-mix-phase), liposome, transfersome, paste or powder.

According to the present invention, the term "auxiliary agent" shall mean any, non-toxic, solid or liquid filling, diluting or packaging material, as long as it does not adversely react and/or interacts with the active ingredients or the patient. Liquid galenical auxiliary agents, for example, are sterile water, physiological saline, sugar solutions, ethanol and/or oils. Galenical auxiliary agents for the production of tablets and capsules, for example, can contain binders and filling materials.

Furthermore, the active ingredients according to the invention can be used in the form of systemically employed medicaments. These include parenterals belonging to which are injectables and infusions. Injectables are either present in the form of ampoules or as so-called ready-to-use injectables, e.g. as ready-to-use syringes or disposable syringes, and, in addition, are provided in puncture-sealed bottles. The administration of the injectables can take place in form of subcutaneous (s.c.), intramuscular (i.m.), intravenous (i.v.) or intracutaneous (i.c.) application. In particular the suitable forms for injection can pe produced as crystal suspensions, solutions, nanoparticular or colloidal-disperse systems, such as, for example, hydrosoles.

The injectable compositions can further be produced as concentrates that are dissolved or dispersed with aqueous isotonic diluents. The infusions can also be prepared in form of isotonic solutions, fatty emulsions, liposome compositions, micro emulsions. Like the injectables, also infusion compositions can be prepared in form of concentrates for dilution. The injectable compositions can also be applied in form of continuous infusions, both in the stationary as well as in the ambulant therapy, e.g. in form of mini pumps.

The active ingredients according to the invention can be bound to a micro carrier or nanoparticle, for example to finely dispersed particles on the basis of poly(meth)acrylates, polylactates, polyglycolates, polyaminoacids or polyetherurethanes. The parenteral compositions can also be modified into a depot preparation, e.g. based on the "multiple unit principle", if an active ingredient according to the invention is embedded in finely divided or dispersed, suspended form or as crystal suspension, or based on the "single unit principle", if an active ingredient according to the invention is included in a medicinal form, e.g. in a tablet or a stick that is subsequently implanted. Often, these implants or depot medicaments in the case of "single unit"- and "multiple unit"-medicaments consist of so-called biodegradable polymers, such as, for example polyesters of lactic and glycolic acid, polyether urethanes, polyaminoacids, poly(meth)acrylates or polysaccharides.

As suitable auxiliary agents for producing of parenterals, aqua sterilisata, substances influencing the value of the pH, such as, for example, organic and inorganic acids and bases as well as their salts, buffer substances for adjusting the value of the pH, isotoning agent, such as, for example, sodium chloride, sodium hydrogen carbonate, glucose and fructose, tensides or surface active substances and emulgators, such as, for example, partial fatty acid esters of polyoxyethylene sorbitane (Tween®) or, for example, fatty acid esters of polyoxyethylene (Cremophor®), fatty oils, such as, for example, peanut oil, soy bean oil, and castor oil, synthetic fatty acid esters, such as, for example, ethyloleate, isopropylmyristate and neutral oil (Miglyol®), as well as polymeric auxiliary agents, such as, for example, gelatine, dextran, polyvinylpyrrolidone, solubility enhancing additives, organic solvents, such as, for example, propyleneglycol, ethanol, N,N-dimethylacetamide, propylenglycole or complex-forming substances, such as, for example, citrate and urea, preservatives, such as, for example, benzoic acid hydroxypropylesters and -methylesters, benzylalcohol, antioxidants, such as, for example, sodiumsulfite and stabilisators, such as, for example, EDTA, can be considered.

In suspensions, the addition of thickening agents in order to avoid the setting of the an active ingredient according to the invention, or the addition of tensides, in order to ensure the admixing of the sediment, or of complex forming agents such as, for example, EDTA is possible. Active ingredient complexes can be achieved with different polymers, such as, for example, polyethyleneglycoles, polystyrenes, carboxymethyl cellulose, Pluronics® or polyethylene glycolsorbite fatty acid esters. For producing lyophilisates, scaffold forming agents, such as, for example, mannit, dextran, sucrose, human albumin, lactose, PVP or gelatines are used.

The medical forms that are each suitable can be produced in accordance with manuals and procedures known to the person of skill on the basis of pharmaceutical/physical technologies.

A further aspect of the present invention then relates to the respectively produced pharmaceutical composition, comprising an effective dose of at least one of a polypeptide according to the present invention, a nucleic acid or vector according to the present invention or an expression vector according to the present invention, a host cell according to the present invention or an antibody according to the present invention, and a pharmaceutically acceptable carrier. This pharmaceutical composition can be characterized in that the active ingredient is present in form of a depot substance or as precursor together with a suitable, pharmaceutically acceptable diluent or carrier substance as above.

According to the present invention, the above pharmaceutical composition can be present in the form of tablets, dragees, capsules, droplets, suppositories, compositions for injection or infusion for peroral, rectal or parenteral use. Such administration forms and their production are known to the person of skill.

In another aspect thereof, the present inventions relates to the use of a polypeptide according to the present invention, a nucleic acid or vector according to the present invention, or an expression vector according to the present invention, a host cell according to the present invention, or an antibody the present invention for detecting a response reaction of a mammal to a silicone. Preferably a response reaction of a human to a silicone.

In another aspect thereof, the present inventions concerns to the use of a polypeptide for detecting a response reaction as described below of a mammal to a silicone, wherein said polypeptide is selected from the group consisting of Myeloid related protein 8, Myeloid related protein 14, HT018, and PRO2619 or a functional variant thereof.

Since the adhesive properties of polypeptides adhering to silicone in different mammals are indicators of a possible response reaction, a diagnostic strategy can be established by identifying polypeptides prone to response reactions and related complications or adverse reactions. Thus, choosing several candidates, e.g. silicone-adhering polypeptides or nucleic acids encoding silicone-adhering polypeptides, a test system for the detection of these polypeptides and/or nucleic acids can be constructed, e.g. by attaching such polypeptides to the surface of silicone from e.g. serum or through amplification methods, such as, for example rt-PCR.

Thereby, polypeptides according to the present invention can be detected with methods known in the art, which comprises, immunological methods like ELISA or Western blotting. Preferably, in one embodiment such test system is based on an ELISA system, a method well-known by a person skilled in the art. Production of ELISA system for the measurement of silicone-adhering polypeptides is easy to perform and stable for a long time at room temperature. This would facilitate shipping to end-users in order to use these tests as "point-of-care" tests, e.g. in the doctor's office. Preferably, such test system uses serum. Moreover, an ELISA system is a standard procedure in many laboratories. This combination would allow for easy integration into current clinical routine.

In another aspect thereof, the present inventions provides a method of diagnostic for detecting a response of a mammal to a silicone, comprising the steps of: a) providing a sample from a mammal to be diagnosed; b) detecting the presence or a change in expression of at least one silicone-adhering polypeptide or a functional variant thereof in the sample; and c) concluding if the presence or change in expression of at least one silicone-adhering polypeptide or a functional variant thereof is a response to the silicone.

In another preferred embodiment of the method of diagnostic for detecting a response of a mammal to a silicone according to the present invention, the silicone-adhering polypeptide is selected from the group consisting of a polypeptide according to the present invention, Myeloid related protein 8, Myeloid related protein 14, HT018, and PRO2619 or a polypeptide identified according by a for identifying polypeptides adhering to silicone surface according to the present invention, or functional variants thereof.

In another preferred embodiment of the method of diagnostic for detecting a response of a mammal to a silicone according to the present invention the silicone-adhering protein and/or polypeptide is detected together with at least one further different silicone-adhering polypeptide selected from the group consisting of Isotype control, IgG-HRP, IgE-HRP, and IGA-HRP, AMBP protein precursor, Plasma-retinol binding protein, Kininogen, Eosinophil peroxidase, Fibroblast growth factor 11, TGF-β, Integrin-β4, T-cell receptor, NADH dehydrogenase, Coagulation factor V, α-spectrin, Monocyte chemotactic protein-2, γ-glutamyltransferase, Immunoglobulin G, Immunoglobulin A, Complement C3, Complement C2, Complement C1s, C reactive protein, Alpha 1-microglobulin/bikunin precursor, Von Willebrandt Factor, Fibronectin, Vitronectin, Fibrinogen, Collagen I, Collagen III, Collagen IV, Collagen VII, Procollagen III, Laminin, Matrixmetalloproteinase 2, Apolipoprotein 4, Apolipoprotein A1, Ceruloplasmin, Thyreotyrein, Albumin, Alpha 2-macroglobulin precursor, Haptoglobin 1, α and β globin chains, Actin, and HSP 60, or functional variants thereof.

In a further preferred embodiment of the method of diagnosis for detecting a response of a mammal to a silicone according to the present invention, the sample from said mammal is serum and/or wound bed fluid.

In another preferred embodiment of the method of diagnostic for detecting a response of a mammal to a silicone according to the present invention sample is collected before or after implantation of a silicone that is suitable for long-term implantations and coating of medical devices.

In a preferred embodiment of the method of diagnostic for detecting a response of a mammal to a silicone according to the present invention, the method further comprises a second sample as a control, wherein said control is either provided from the same mammal before or after implantation, or from a different mammal without any implantations of a medical silicone.

In another preferred embodiment of the method of diagnostic for detecting a response of a mammal to a silicone according to the present invention, for detecting silicone-adhering polypeptides or a functional variants thereof antibodies are used suitable to immunologically recognize said polypeptide or functional variant thereof.

In a further preferred embodiment of the method of diagnostic for detecting a response of a mammal to a silicone according to the present invention, the polypeptide or functional variant thereof are detected by one-or two-dimensional gel electrophoresis and/or ELISA.

In a preferred embodiment of the method of diagnostic for detecting a response of a mammal to a silicone according to the present invention, the method further comprises detecting the presence or amount of at least one nucleic acid having a nucleotide sequence encoding for a silicone-adhering polypeptide according to the present invention.

In another preferred embodiment of the method of diagnostic for detecting a response of a mammal to a silicone according to the present invention, said nucleic acid is detected by PCR, RT-PCR or gel electrophoresis.

In a further preferred embodiment of the method of diagnostic for detecting a response of a mammal to a silicone according to the present invention, said method further comprises between step a) and b) step a') of contacting said sample with a silicone, whereby the proteins are adhered to the silicone.

In another aspect thereof, the present inventions relates to a diagnostic kit for detecting a response reaction in a mammal comprising materials, buffers, auxiliary agents, and substances for performing a method of diagnosis for detecting a response of a mammal to a silicone according to the present invention.

The term "diagnostic kit" as used herein, shall mean a reagent kit comprising a polypeptide, a nucleic acid molecule, a vector, a host cell, or an antibody of the present invention or as identified or characterized by a method of the present invention. Preferably the reagent kit further comprises reaction buffer(s), storage solutions and/or remaining reagents or materials required for the conduct of scientific or diagnostic assays or the like. Furthermore, parts of the kit of the invention can be packed individually in vials or bottles or in combination in container or multi-container units.

In another aspect thereof, the present inventions relates to a method for monitoring a response reaction of a mammal to a silicone, by detecting the presence or a change in expression of a silicone-adhering polypeptide by a method or a reagent kit for detecting a response of a mammal to a silicone according to the present invention.

In yet another aspect thereof, the present inventions concerns a method for screening substances interfering with a silicone-adhering polypeptide, comprising the steps of a) providing a substance, wherein the substance is suspected to interfere with the adhering properties of a silicone-adhering polypeptide; b) providing of a silicone; c) providing a silicone-adhering polypeptide; and d) analyzing the interfering properties of the substance through analyzing adhering of the silicone-adhering polypeptides to the silicone in the presence or absence of the substance.

In a embodiment of method for screening active substances interfering with a silicone-adhering protein and/or polypeptide according to the present invention the silicone-adhering polypeptide is selected of a group consisting of a polypeptide according to the present invention, Myeloid related protein 8, Myeloid related protein 14, HT018, PRO2619, a polypeptide identified according to the present invention, Isotype control, IgG-HRP, IgE-HRP, and IGA-HRP, AMBP protein precursor, Plasma-retinol binding protein, Kininogen, Eosinophil peroxidase, Fibroblast growth factor 11, TGF-β, Integrin-β4, T-cell receptor, NADH dehydrogenase, Coagulation factor V, α-spectrin, Monocyte chemotactic protein-2, γ-glutamyltransferase, Immunoglobulin G, Immunoglobulin A, Complement C3, Complement C2, Complement C1s, C reactive protein, Alpha 1-microglobulin/bikunin precursor, Von Willebrandt Factor, Fibronectin, Vitronectin, Fibrinogen, Collagen I, Collagen III, Collagen IV, Collagen VII, Procollagen III, Laminin, Matrixmetalloproteinase 2, Apolipoprotein 4, Apolipoprotein A1, Ceruloplasmin, Thyreotyrein, Albumin, Alpha 2-macroglobulin precursor, Haptoglobin 1, α and β globin chains, Actin, and HSP 60, or functional variants thereof.

In another aspect thereof, the present invention provides a method for detecting cell adhesion to silicone, comprising a) providing a sample from a mammal; b) providing of silicone suitable for long-term implantation and coating of medical devices; and c) analyzing the amount or number of adhered cells.

In a preferred embodiment of the method for detecting cell adhesion to silicone according to the present invention, the sample is serum or wound bed fluid of a mammal, preferably of a human.

In a preferred embodiment of said method for detecting cell adhesion to silicone according to the present invention the silicone can be any medical silicone known by the person skilled in the art. Preferably, the silicone is selected from the group comprising medical silicone types MED1511, MED4860, and MED4211 provided by the company Nusil (Waldbroon, Germany).

In another aspect thereof, the present inventions provide a method of treating a mammal suffering from a response reaction to an implanted silicone, by administering of an effective amount of a substance identified through a method for screening active substances interfering with a silicone-adhering polypeptide according to the present invention together with a suitable pharmaceutical carrier to a mammal. Preferably said effective amount of a substance interfering with a silicone-adhering polypeptide is administered in the form of a pharmaceutical composition, those compositions are well known by a person skilled in the art.

In a preferred embodiment of the method of treating a mammal suffering from a response reaction to an implanted silicone according to the present invention, the response reaction is a hypersensitivity reaction to an implanted silicone.

In another aspect thereof, the present inventions relates to a method for monitoring the treatment of a mammal suffering from a response reaction to an implanted silicone, comprising: a) administering a substance interfering with the adhering properties of a silicone-adhering polypeptide; b) obtaining a sample from the mammal; c) detecting the presence or a change in expression of at least one silicone-adhering polypeptide or a functional variant thereof; and d) reducing or increasing the amount of the substance interfering with the adhering properties of a silicone-adhering polypeptide administered to the mammal in respect to the presence or the change in expression of at least one silicone-adhering polypeptide or a functional variant thereof.

In a preferred embodiment of the method for monitoring the treatment of a mammal suffering from a response reaction to an implanted silicone according to the present invention, the sample is serum or wound bed fluid of a mammal, preferably of a human.

In a preferred embodiment of the method for monitoring the treatment of a mammal suffering from a response reaction to an implanted silicone according to the present invention the silicone-adhering polypeptide is selected of a group consisting of a polypeptide according to the present invention, Myeloid related protein 8, Myeloid related protein 14, HT018, PRO2619, a polypeptide identified according to the present invention, Isotype control, IgG-HRP, IgE-HRP, and IGA-HRP, AMBP protein precursor, Plasma-retinol binding protein, Kininogen, Eosinophil peroxidase, Fibroblast growth factor 11, TGF-β, Integrin-β4, T-cell receptor, NADH dehydrogenase, Coagulation factor V, α-spectrin, Monocyte chemotactic protein-2, γ-glutamyltransferase, Immunoglobulin G, Immunoglobulin A, Complement C3, Complement C2, Complement C1s, C reactive protein, Alpha 1-microglobulin/bikunin precursor, Von Willebrandt Factor, Fibronectin, Vitronectin, Fibrinogen, Collagen I, Collagen III, Collagen IV, Collagen VII, Procollagen III, Laminin, Matrixmetalloproteinase 2, Apolipoprotein 4, Apolipoprotein A1, Ceruloplasmin, Thyreotyrein, Albumin, Alpha 2-macroglobulin precursor, Haptoglobin 1, α and β globin chains, Actin, and HSP 60, or functional variants thereof.

The present invention shall now be described further in the following examples with respect to the attached drawings without being limited thereto. For the purposes of the present invention, all references as cited herein are incorporated by reference in their entireties.

### BRIEF DESCRIPTION OF THE DRAWINGS

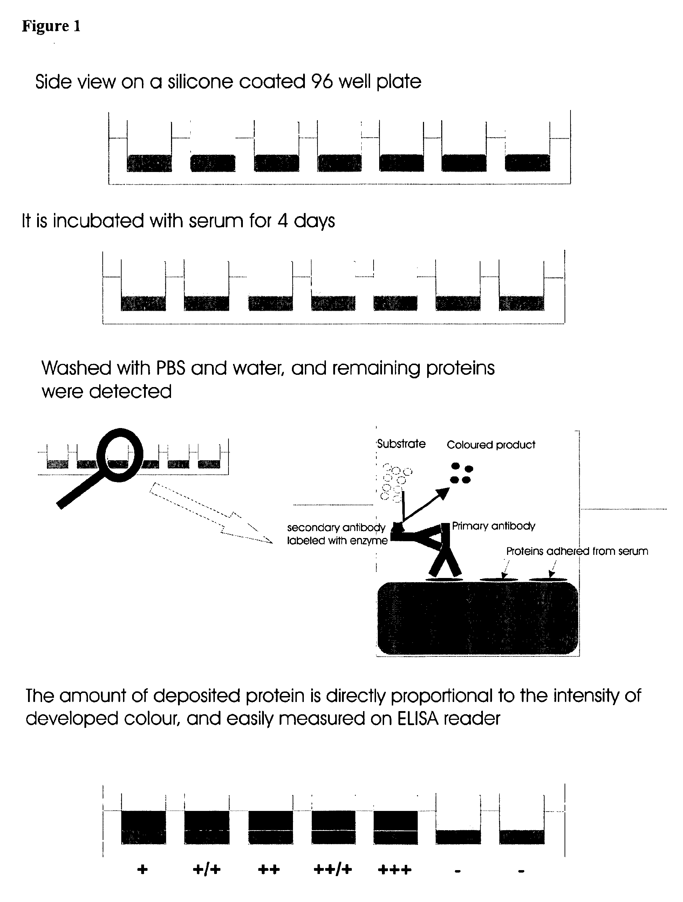

**Figure 1.** Construction scheme of the ELISA system for protein adhesion to silicone. A 96-well plate was coated with silicone and sterilized as described. It was then incubated with serum samples (complemented with protease inhibitors) for 4 days and washed with PBS and water. Adhering proteins were detected using specific primary antibodies and appropriate secondary antibodies labeled with enzyme (e.g. horseradish peroxidase) converting uncolored substrate into a colored product that was measured at a specific wavelength. The amount of protein was directly proportional to the intensity of the developed dye. The assay can be performed in various other formats.

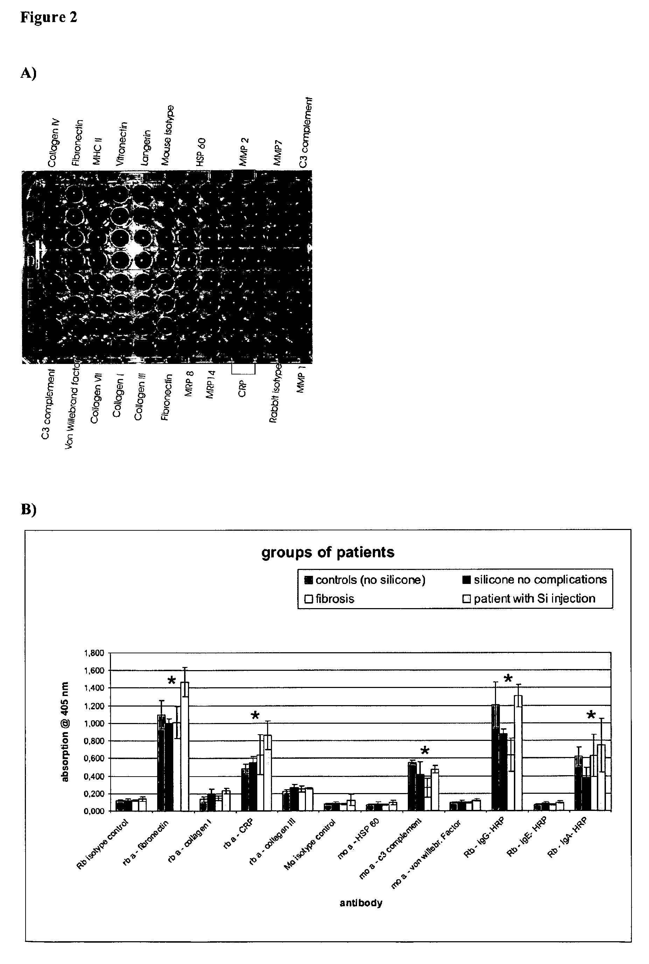

**Figure 2.** Proof of principle screen of patients with fibrotic complications upon implantation of SMIs. Following the ELISA principle as shown in Figure 1, 12 protein candidates in 10 patients (5 with and 5 without fibrotic complications), 7 controls (age and sex matched) and one patient with fluid silicone injection causing dramatic generalized fibrotic complications were tested. (A) Photo of a plate with protein candidates tested. Green coloration indicated high abundance of protein. The variations in protein deposition could generally be distinguished macroscopically. However, absorption measurements of each well were performed for exact quantification and statistical analysis of results. (B) Statistical analysis of different patient groups. After the absorption was measured, values were expressed as means ± standard deviations for each group. The Kolmogorov-Smirnoff test was used to test the normality of the distribution, and t-test was used to calculate p-values. A significant positive correlation of the occurrence of a fibrotic complication with the deposition of fibronectin 1, C-reactive protein (CRP) and IgA, and significant negative correlation with the deposition of complement component C3 and IgG was found.

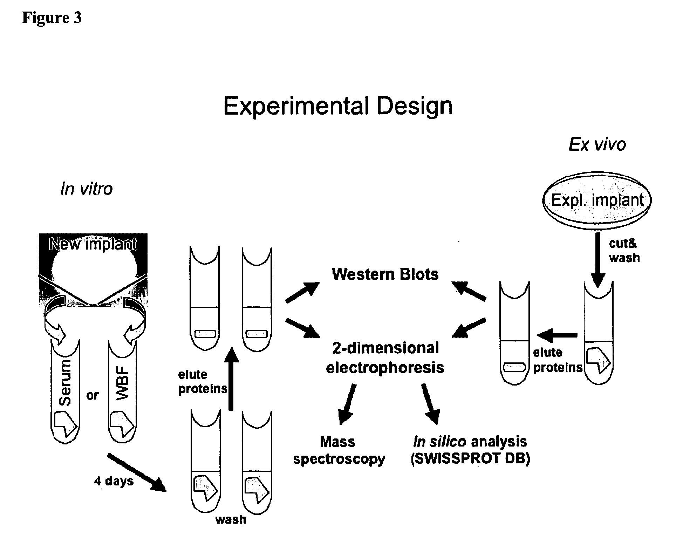

**Figure 3.** Algorithm of *ex vivo* and *in vitro* protein adhesion analysis. In *ex vivo* analysis saline-filled explanted SMIs were cut in pieces, washed, and adhering proteins eluted in a buffer appropriate for downstream analysis (either for Western blot or for 2D electrophoresis/MS). *For in vitro* analysis standardized pieces of silicone cut out of new, intact sterile saline-filled SMIs were incubated for 4 days with WBF or serum. They were then washed in exactly the same way as *ex vivo* prepared SMIs, and proteins eluted in an appropriate buffer for downstream analysis (either for Western blot or for 2D electrophoresis/MS). For comparison, all protein amounts were normalized per unit of surface.

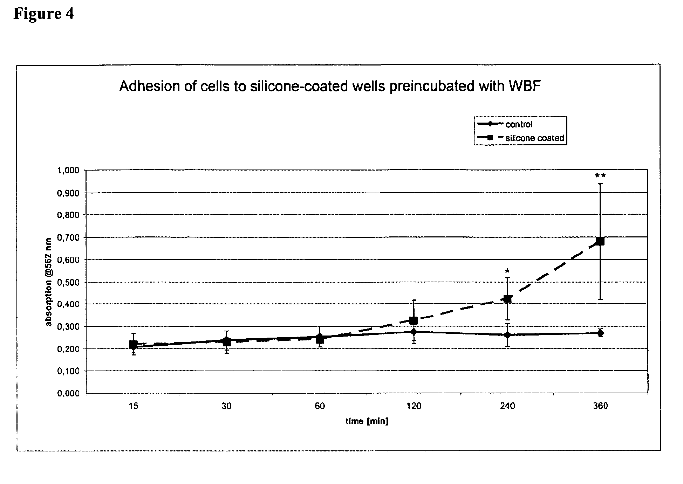

**Figure 4.** Adhesion of foreskin fibroblasts to silicone coated with serum proteins. Human primary fibroblasts were grown out from foreskin preparations. After their phenotype homogeneity had been determined (data not shown), they were cultured under standard conditions. 96-well plates were coated with silicone, while uncoated polystyrene plates were taken as control. Both were incubated with serum for 4 days and extensively washed with PBS and water. Fibroblasts were then seeded onto both silicone-coated and uncoated plastic wells and eluted at various time points between 15 and 300 minutes. Stronger adhesion of fibroblasts to silicone-coated than to uncoated wells (statistically significant after 240 min *p<0.05, and highly significant after 300 minutes **p<0.001) could be observed. This indicated that preferential adhesion of protein from serum to silicone promoted fibroblast adhesion.

### EXAMPLES

### Example 1: Construction of an in vitro system for screening of proteins adhering to various silicone types in vivo

The principle of protein preparation and analysis is shown in Figure 3.

### 1. Proteins eluted ex vivo

The inventors have analyzed proteins deposited on SMIs explanted from women suffering from complications after breast augmentation for cosmetic reasons. Thirteen explanted SMIs were obtained from the Clinic for Plastic and Reconstructive Surgery, Medical University Innsbruck (MUI, Innsbruck, Austria). Nine SMIs were gel and 4 saline filled. Eleven SMIs were rough and 2 smooth surfaced. Only intact implants, i.e. without silicone 'gel bleeding', were considered for analysis. They were taken under sterile conditions immediately after surgery. The fibrotic capsule was removed in the surgical theatre, and SMIs were transported to the laboratory in phosphate buffered saline (PBS, pH 7.2) supplemented with protease inhibitors. Explanted SMIs were washed with PBS (3x15min) and distilled water (5x10min) at 4°C. After washing, proteins were eluted either in urea lysis buffer [7M urea, 2M thiourea (both from Calbiochem, Bad Soden, Germany), 30mM TRIS, 4% CHAPS, pH 8.5], if they were going to be analyzed by 2D electrophoresis. For Western blot analysis, they were prepared in SDS buffer (2%SDS, 100mM DTT, 50mM TrisCl, 10% Glycerol, pH 9.0). Protein concentration was measured by the bicinchoninic acid protein assay (Pierce Biotechnology: Rockford, IL, USA), and protein solutions were stored frozen at -20°C until analysis.

### 2. Proteins eluted in vitro

As silicone types MED1511, MED4860 and MED4211 (NUSIL; Waldbroon, Germany) do not contain or require organic solvents or high temperatures for polymerization, they were tested first. Their physical characteristics made them compatible with polystyrene flat bottom plates routinely used in laboratory practice that we employed in order to have controlled and standardized conditions. All silicone types tested were unrestricted types, i.e. they were certified from producer for long-term implantation by the silicone manufacturer (Nusil; Waldbronn, Germany). They also represented silicone types that are widely used in SMI manufacturing and coating of medical devices. Other silicone types are planned by the inventors to be tested in the future, and the design of the test will be adjusted according to their chemical composition.

Five ml of unpolymerized silicone was added per well of 6 well polystyrene plates (Greiner, Frickenhausen, Germany), and plates were immediately centrifuged for various times, depending on the silicone type (MED 1511: 10 min at 300g; MED 4860: 60 min at 1000g; MED 4211: 10 min at 150g) in order to distribute silicone evenly on the bottom of the plate. After silicone curing (3 days at 37°C and 98% humidity), plates were sterilized for 120 seconds under 200 mJoules of UV light (GsGene UV chamber, Biorad, Hercules, CA) and incubated with wound bed fluid (WBF) taken 2 days after breast augmentation surgery in order to allow for saturation of protein binding sites in the tubing. WBF was supplemented with protease inhibitors (pepstatin, leupeptin, aprotinin from Sigma at final concentration 1 µg/ml) and incubated with silicone coated plates for 4 days. Within this time, the equilibrium of protein deposition on the surface of silicone was reached while no spontaneous protein degradation occurred. Plates were then washed (10x15min) in PBS and (5x10 min) distilled H₂O at 4°C, and proteins eluted in the same way as the *ex vivo* protein elution described above. Figure 3 shows the model for *in vitro* protein adsorption and analysis.

### 3. Differential analysis of proteins eluted ex vivo and in vitro

To determine, if the *in vitro* model corresponds to the situation *ex vivo,* the inventors compared proteins eluted from the two systems by one and two-dimensional gel electrophoresis. For differential analysis, the inventors needed a sensitive analytical tool and that is why the DIGE^{™} (Amersham Biosciences, Upsala Sweden) system was employed. Proteins from the two systems were labelled with different fluorochromes that can be excited at specific wavelengths. This experimental setup allowed the inventors to obtain three independent images from a single gel, greatly reducing gel to gel variation and enhancing statistical analysis of the spot patterns. Protein spot patterns were analyzed with the 'Decyder' software (Amersham Biosciences). The amount of proteins deposited was considered different, if the spot volume ratio between samples exceeded two standard deviations of total spot distribution. More stringently, it was defined as present only in either the *ex vivo* or the *in vitro* system, if the volume ratio exceeded a threshold of fivefold. Using this approach, the inventors detected so far a total of 184 protein spots. Of those, 163 spots were present in the same amounts in both systems, 13 proteins bound stronger in the *ex vivo* and 5 in the *in vitro* system, and only 5 spots were unique for either of the systems. In other words, 89% of protein deposited *in vivo* are also deposited in our *in vitro* system. Using mass spectrometry, the inventors identified most of the remaining 11% of proteins and found that they mainly represented intracellular proteins. These findings suggested that additional proteins found *in vivo* originated from the surrounding damaged tissue as a consequence of the surgical procedure.

### 4. Protein identification

### 2D electrophoresis

Proteins eluted *in vitro* and *ex vivo* were primarily analyzed after separation by two-dimensional gel electrophoresis (2D electrophoresis) [Anderson, L. and Anderson, N. G. High resolution two-dimensional electrophoresis of human plasma proteins. Proc. Natl. Acad. Sci. U. S A 74, 5421-5425 (1977)]. For the first dimension separation, proteins were dissolved in urea lysis buffer [7M urea, 2M thiourea (both from Calbiochem, Bad Soden, Germany), 30mM TRIS, 4%CHAPS, pH 8.5]. Prior to loading, 200µg of total protein was supplemented with rehydration buffer (7M urea, 2M thiourea, 4% CHAPS, 18mM DTE) to a final volume of 350 µl. The inventors then added resolyte buffer and loaded proteins on to 18 cm IPG strips pI 3-11. Proteins were focused for a total of 55000 Vhrs (IPGphor, Amersham Biosciences). After isoelectric focusing, proteins on strips were reduced (2% DTT, 15 min) and subsequently alkylated (2.5 % IAA, 15 min). Second dimension electrophoresis was performed under reducing conditions, in 20x24 cm gradient 9-16% polyacrylamide gels at 16°C. After 2D electrophoresis, gels were fixed and silver stained according to the Farmer's procedure [Wray, W., Boulikas, T., Wray, V. P., and Hancock, R. Silver staining of proteins in polyacrylamide gels. Analytical Biochemistry 118, 197-203 (1981)].

### Pattern matching and mass spectrometry

Silver stained protein spot patterns were matched and compared to the ExPasy database of 2D gels (http://us.expasy.org/ch2d/) using the 'Melanie' software (version 4, Swiss Institute of Bioinformatics, Basel, Switzerland) [Hughes, G. J., Frutiger, S., Paquet, N., Ravier, F., Pasquali, C., Sanchez, J. C., James, R., Tissot, J. D., Bjellqvist, B., and Hochstrasser, D. F. Plasma protein map: an update by microsequencing. Electrophoresis 13, 707-714 (1992)]. Spots of interest were manually excised, proteins digested with trypsin (Promega, Mannheim, Germany), and peptides analyzed by Mass spectrometry (Ultraflex TM TOF/TOF, BrukerDaltonics, Billerica, MA).

In gel digestion was performed as described by Shevchenko et al. [Shevchenko, A., Wilm, M., Vorm, O., and Mann, M. Mass spectrometric sequencing of proteins silver-stained polyacrylamide gels. Analytical Chemistry 68, 850-858 (1996)]. Those samples which were analyzed by peptide mass fingerprint (PMF) from MALDI-TOF were additionally analyzed using LIFT-TOF/TOF MS/MS from the same target. A maximum of three precursor ions per sample were chosen for MS/MS analysis. PMF was measured with reflector mode, followed MALDI-TOF/TOF measurement with LIFT mode. PMF spectra were interpreted with the 'Mascot' and 'Profound' software. The probability score higher than 64 for 'Mascot' and 1.65 for 'Profound' was used as a cut-off criterion for the correct identification.

### Confirmation of results with Western Blots

To complement and confirm the data obtained from mass spectroscopy and database search, the inventors performed Western blots of proteins eluted from silicone. For this purpose, the inventors used proteins eluted in SDS buffer (2%SDS, 100mM DTT, 50mM TrisCl, 10% glycerol, pH 9.0) that were loaded to small (7x10 cm) homogenous 12% polyacrylamide gels. After the run, proteins were blotted to on the nitrocellulose membrane for 1200 mAh in a wet blotting chamber, and blocked with 5% fat free milk. Primary antibodies against most of the proteins, as well as appropriate conjugates, were obtained from DAKO, polyclonal rabbit antibodies against Myeloid Related Protein (MRP) 8 and MRP 14 were a kind gift from Dr. Claus Kerkhoff (Institute of Experimental Dermatology; Muenster, Germany), and we ourselves produced affinity chromatography purified monoclonal antibodies against human HSP60, from clone II-13 [Xu, Q., Schett, G., Seitz, C. S., Hu, Y., Gupta, R. S., and Wick, G. Surface staining and cytotoxic activity of heat-shock protein 60 antibody in stressed aortic endothelial cells. Circ. Res. 75, 1078-1085 (1994)]. All antibody dilutions were prepared in 5% fat free milk in PBS. After the secondary antibody incubation membranes were washed with PBS and chemiluminescent substrate for horseradish peroxidase (Amersham Biosciences) was added for 1 min. Membranes were exposed to AGFA x-ray films for various times and developed in the Currix developing machine.

### Example 2: Profile of proteins differentially adhering to silicone

Proteins found adhering both *ex vivo* and in *vitro* to different selected medical silicone types are summarized in Table 1. Their relative abundance is expressed in the following way: ?: not tested, 0: absent, +: present, ++: abundant, +++: highly abundant.

Proteins could be classified into different categories: anchor proteins, a primary protein set, and a secondary protein set. Only the first two categories have been reconfirmed by alternative techniques (Western Blot, ELISA).

### 1. Proteins of equal, strong adhesion (anchor proteins)

These proteins were defined as at least 'abundant', i.e. ++, and presented as equally adhesive towards all silicone types tested. Seven (7) candidates were included into this group. They are coloured in red.

### 2. Primary protein set

This set consisted of twenty-one (21) proteins. For inclusion they had to be detected in all of the tested silicone types. Three subcategories could be defined:

### Proteins of equal, but low adhesion

Fourteen proteins (14) constituted this subcategory, which was defined as 'present', i.e. +. They are labeled in blue.

### Proteins of differential silicone type adhesion

Seven (7) proteins showed a significant difference in adhesion to the silicone types MED1511, 4860 and 4211 (Nusil). They covered molecules of all adhesion strength, i.e. 0, +, ++ and +++. They are in orange.

### Proteins of unclassified adhesion properties

This group of four (4) proteins was tested on two silicone types only. The successful experiments showed adhesion class 'present' (+). So far they would most likely be members of subcategory 1 (proteins with equal, but low adhesion). These candidates are in black.

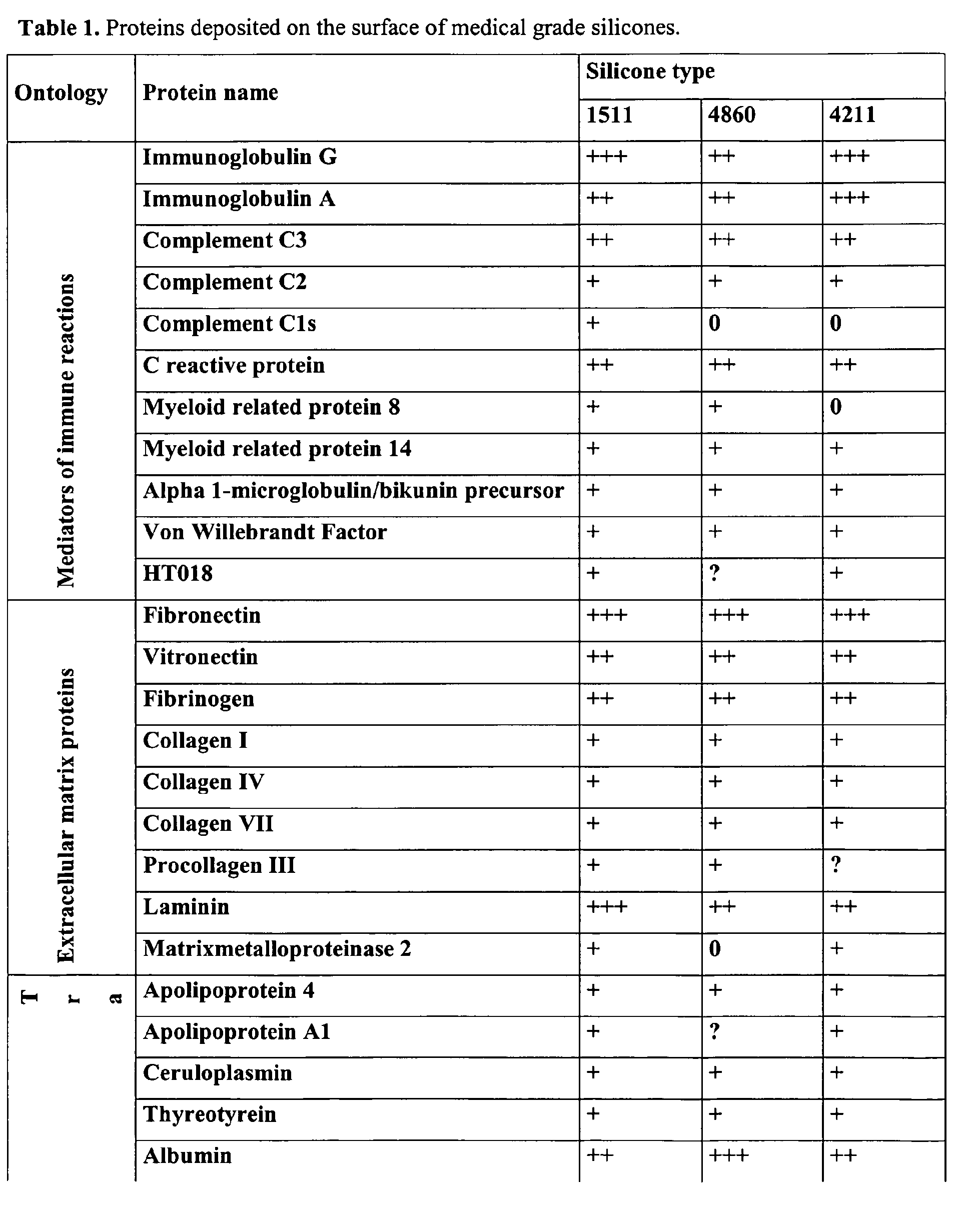

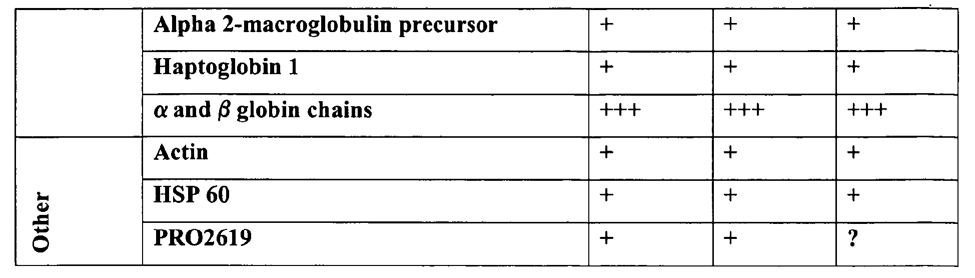

### 3. Secondary protein set

This set consisted of seventeen (17) proteins. They were detected on one silicone type only (MED1511) and have not been confirmed by techniques other than mass spectrometry.

### Annotated proteins

The following thirteen (13) proteins were part of this category:
AMBP protein precursor
Plasma-retinol binding protein
Kininogen
Eosinophil peroxidase
Fibroblast growth factor 11
TGF-β
Integrin - β4
T-cell receptor
NADH dehydrogenase
Coagulation factor V
α-spectrin
Monocyte chemotactic protein-2
γ-glutamyltransferase

### Not annotated proteins

There were four (4) unknown *Homo sapiens* protein products and hypothetical proteins as identified by 'Masscot':
Acc. no.: gi|34533774; mass: 113642; unknown protein product (SEQ ID NO. 1)
Acc. no.: gi|21739298, emb|CAD38696.1; mass: 117490; unknown protein product (SEQ ID NO. 2)
Acc. no.: gi|21754778; mass: 38453; unknown protein product (SEQ ID NO. 3)
Acc. no.: gi|7020569; mass: 14004; unknown protein product (SEQ ID NO. 4)

### 3. Pending protein set

Ten (10) more proteins are still pending for MS analysis.

### Example 3: Novel protein candidates

Among the proteins mentioned above there were several ones, which so far have not been mentioned in connection with hypersensitivity to silicone and consecutive fibrosis:
Myeloid related protein 8
Myeloid related protein 14
HT018
PRO2619
Acc. no.: gi|34533774; mass: 113642; unknown protein product (SEQ ID NO. 1)
Acc. no.: gi|21739298, emb|CAD38696.1; mass: 117490; unknown protein product (SEQ ID NO. 2)
Acc. no.: gi|21754778; mass: 38453; unknown protein product (SEQ ID NO. 3)
Acc. no.: gi|7020569; mass: 14004; unknown protein product (SEQ ID NO. 4)

### Example 4: Diagnostic test system

It is hypothesized that the adhesion properties of proteins adhering to silicone in different patients are indicators of the possible immune and subsequent fibrotic reactions individuals might have developed. If this hypothesis was correct, the inventors could establish a diagnostic strategy, which identified proteins prone to fibrotic complications. Thus, after having performed protein analyses (see Example 2 and 3), the inventors chose several candidates and constructed a test system for the detection of these proteins recruited to the surface of silicone from serum.

### Practical advantages

1) The test protocol is based on ELISA microwell plates. Production of this test system is easy to perform and stable for a long time at room temperature. This would facilitate shipping to end-users.
2) The test system uses sera. Moreover, an ELISA system is a standard procedure in many laboratories. This combination allows an easy integration into current clinical routine.

### 1. Technical details

Polystyrene 96 well flat bottom microtiter plates were coated with medical silicone types 1511, 4860 and 4211. Fifty µl of unpolymerized silicone was added per well of 96-well polystyrene plates (Greiner, Frickenhausen, Germany), and plates were immediately centrifuged for various times, depending on the silicone type (MED 1511: 10 min at 300g; MED 4860: 60 min 1000g; MED 4211: 10 min-150g) in order to distribute silicone evenly on the bottom of the plate. After silicone curing (3 days at 37°C and 98% humidity), plates were sterilized for 120 seconds under 200 mJoules of UV light (GsGene UV chamber, Biorad, Hercules, CA). Following sterilization, silicone coated 96 well plates were incubated with serum from patients before and after undergoing primary breast augmentation or with serum of patients having their SMIs exchanged. All sera are supplemented with 1 µg/ml of leupeptin, pepstatin and aprotinin (protease inhibitors, Sigma-Aldrich, Vienna, Austria), filtered through 0.22 µm filters and 100 µl is applied to each well under aseptic conditions. In some experiments, sera were diluted with PBS prior to application. After 50 hours, excess proteins were washed away with 4x 5 min washing with PBS, and further binding sites on the plates blocked with 2.5% BSA/0,1%tween 20 in PBS for 100 minutes at room temperature. One hundred and fifty µl of blocking solution were applied per well. Following blocking, various antibodies were applied in previously determined optimal dilutions in blocking solution. Our initial screen was composed of 24 antibodies, but we reduced our current list to 12 applied in the following optimal working dilutions (Table 2):

**Table 2. Antibodies used for the modified ELISA system for protein adhesion.**

| **Column** | **Antibody against** | **From** | **Dilution** | **Company, serial No.** |
|---|---|---|---|---|
| 1 | isotype control | Mouse | 1:1000 | DAKO X0931 |
| 2 | Isotype control | Rabbit | 1:1000 | DAKO X0903 |
| 3 | Fibronectin | Rabbit | 1:1000 | DAKO A245 |
| 4 | Collagen I | Rabbit | 1:500 | Calbiochem 234167 |
| 5 | CRP | Rabbit | 1:500 | DAKO A0073 |
| 6 | HSP 60 | Mouse | 1:1000 | Supernat. clone II-13 |
| 7 | C3 complement | Mouse | 1:500 | DAKO M0836 |
| 8 | Von Willebrand factor | Mouse | 1:500 | DAKO M0616 |
| 9 | Collagen III | Rabbit | 1:500 | Chemicon AB 747 |
| 10 | IgG - HRP | Rabbit | 1:500 | DAKO P 214 |
| 11 | IgE - HRP | Rabbit | 1:500 | DAKO P 295 |
| 12 | IgA - HRP | Rabbit | 1:500 | DAKO P 216 |

It is important to state here that this list of antibodies is, of course, not a final one. It will grow according to the new findings the inventors obtain in the protein analysis of the film formed on the surface of silicone. After incubation with primary antibody, the plate was washed 3x5 min PBS, and appropriate secondary antibodies conjugated with HRP were applied for 120min, in 100 µl/well volume, on a shaking platform at room temperature. Wells incubated with directly labelled primary antibodies were kept in PBS until the ABTS development step. The following secondary antibodies were used: Rabbit anti-mouse IgG HRP, DAKO P260, 1:1000 and swine anti-rabbit IgG HRP, DAKO P399, 1:1000. All dilutions were made in blocking solution. After the secondary antibody incubation at room temperature, the plate was washed 3x5 min in PBS/0.02% Tween, and 2 x 5min in pure PBS. The standard ABTS ELISA detection solution for HRP was used to visualize the results. Color was developed for 40 min, the solution moved to new 96 well plates (to exclude silicone coating interference with spectrophotometric measurement), and the absorption measured at 405 nm. Thus, we have constructed an ELISA system for the measurement of protein adhesion to silicone (Figure 1).
Titrating different blocking solutions, the inventors managed to significantly reduce the background level. The inventors defined the cut-off for presence of a protein as 2 absorbance values of isotype controls. Background values were less than 1% of probe measurements, the linear range spanning over two orders of magnitude. The whole test system was highly reproducible and allowed for semi-quantitative analysis of the deposited proteins. A major advantage of this system is that simultaneous analysis of large number of patients and various proteins respectively deposited onto silicone is possible in a relatively short time.

As a proof of principle (Figure 2), the inventors tested 12 protein candidates in 10 patients (5 with and 5 without fibrotic complications), 7 controls (age and sex matched) and one patient with fluid silicone injection (!) causing dramatic generalized fibrotic complications. We found a significant positive correlation of the deposition of fibronectin, CRP and IgA with fibrosis, and negative correlation with the deposition of complement component C3 and IgG.

Thus, the inventors have noticed different pattern of protein deposition from various patients as a proof of principle. In fact, the inventors would like to extend the analysis and correlate the results with implant duration and local or systemic complications on a large cohort of patients. For this purpose, the inventors have adjusted the test system to a 384-well format, which allows for rapid screening and larger clinical studies. Moreover, the amount of sera, which often is a limiting factor for the analysis, can be reduced, saving costs as well as enhancing reproducibility due to the possibility of replication. The detection system with primary antibodies against specific proteins and appropriate HRP conjugated secondary antibodies is the same as above, but in lower volume (50µl). Colour was developed for 40 min, the solution moved to new 384 well plates (to exclude silicone coating interference with spectrophotometric measurement), and the absorption measured at 405 nm on "Victor" 384 well plate spectrophotometer (Perkin Elmer, Boston, USA).

### Example 5: System for testing drugs

Preferential adhesion of selected protein candidates to silicone suggests their functional relevance for the development of the hypersensitivity reaction and the associated fibrotic complication. Consequently, one could try to interfere with protein attachment by using drugs, which either covered the adhesion sites for these proteins or captured the candidates before they bound to the silicone surface. Thus, the inventors recommend to use the following test system for a future drug screening application. It is based on polystyrene 384-microwell plates. The principle of silicone coating is almost identical as in 2.4.1.2. The only difference is that 10µl of each unpolymerized silicone was applied per well instead of 50. The amount of serum or any other protein solution applied per well is also significantly smaller, and it is reduced to 20 µl per well. All incubation times remain the same.

### Example 6: In vitro cell adhesion to silicone

The same system of silicone coated plates was used for cell adhesion experiments. As described above, plates were coated with various silicone types, and either incubated or not with WBF or serum for 4 days. After this period, plates were washed, and 2 ml of 5x10⁴ cells /ml suspension in DMEM medium without serum were added per well. At various time points, the nonadherent cells were removed by gentle pipetting, and the remaining cells were fixed in 4% formaldehyde. The relative number of attached cells was determined in a crystal violet assay [Gailit, J., Clarke, C., Newman, D., Tonnesen, M. G., Mosesson, M. W., and Clark, R. A. Human fibroblasts bind directly to fibrinogen at RGD sites through integrin alpha(v)beta3. Experimental Cell Research 232, 118-126 (1997)]. Crystal violet solution was added (0.2% w/v in 2% ethanol), and incubated for 10 minutes at room temperature. Plates were washed two times in tap water by immersion in a large beaker. Then they were drained upside down on paper towels, and 1% SDS was added to solubilize the stain. Plates were agitated on an orbital shaker until the colour was uniform with no areas of dense coloration in bottom of wells, and absorbance of 1% SDS solution from each well was read at 562 nm, with 1% SDS as a blank. The solution was measured by classical spectrophotometry. The whole test could be performed on six-well plates and downsized to a 96-well format for high throughput application. Figure 4 shows fibroblast adhesion to the silicone coated plastic wells.

## Claims

1. A method for identifying polypeptides adhering to a silicone surface, comprising the following steps of:
a) providing a sample from a mammal, wherein said sample is suspected to contain silicone-adhering polypeptides;
b) optionally, providing said sample with a silicone;
c) eluting of polypeptides that are adhered to said silicone in said sample; and
d) identifying said eluted polypeptides.

2. The method according to claim 1, wherein said sample is selected from a proteinaceous film covering said silicone, wound bed fluid, and serum from a mammal showing a response reaction to implanted silicone.

3. The method according to claim 1 or 2, further comprising step e) further identifying polypeptides that are suitable as marker polypeptides for detecting a response reaction in a mammal to silicone.

4. The method according to claim 3 wherein step e) comprises:
e') comparing polypeptide patterns derived from the separation of said proteins eluted from the proteinacous film covering said silicone with polypeptide patterns derived from the separation of silicone-adhering polypeptides eluted from wound bed fluid or serum; and
e") selecting and analyzing a spot of interest in said pattern.

5. A reagent kit for identifying polypeptides adhering to a silicone surface, comprising materials, buffers and auxiliary agents and substances for performing the method according to any of claims 1 to 4.

6. A silicone-adhering polypeptide identified through a method according to any of claims 1 to 4.

7. Polypeptide comprising an amino acid sequence selected from the group consisting of SEQ ID NO. 1, SEQ ID NO. 2, SEQ ID NO. 3, and SEQ ID NO. 4 or a functional variant thereof, wherein said polypeptide or the functional variant thereof adheres to the surface of a silicone.

8. A nucleic acid comprising a nucleotide sequence encoding for a polypeptide according to claim 6 or 7, or a functional variant thereof, or a complementary nucleotide sequence of said nucleic acid.

9. Use of a polypeptide for detecting a response reaction of a mammal to a silicone, wherein said polypeptide is selected from the group consisting of SEQ ID NO. 1, SEQ ID NO. 2, SEQ ID NO. 3, SEQ ID NO. 4, Myeloid related protein 8, Myeloid related protein 14, HT018, and PRO2619 or a functional variant thereof.

10. A method of diagnosis for detecting a response reaction of a mammal to a silicone, comprising the steps of:
a) providing a sample from a mammal to be diagnosed;
b) detecting the presence or a change in expression of at least one silicone-adhering polypeptide or a functional variant thereof in said sample; and
c) concluding from said presence or change in expression of said at least one silicone-adhering polypeptide or a functional variant thereof on a response reaction to said silicone.

11. The method according to claim 9, wherein said silicone-adhering polypeptide is selected from the group consisting of SEQ ID NO. 1, SEQ ID NO. 2, SEQ ID NO. 3, SEQ ID NO. 4, Myeloid related protein 8, Myeloid related protein 14, HT018, and PRO2619 or a polypeptide identified according to a method according to any of claims 1 to 4, or a functional variant thereof.

12. The method according to claim 10 or 11, wherein said silicone-adhering polypeptide is detected together with at least one further different silicone-adhering protein and/or polypeptide selected from the group consisting of Isotype control, IgG-HRP, IgE-HRP, and IGA-HRP, AMBP protein precursor, Plasma-retinol binding protein, Kininogen, Eosinophil peroxidase, Fibroblast growth factor 11, TGF-β, Integrin-β4, T-cell receptor, NADH dehydrogenase, Coagulation factor V, α-spectrin, Monocyte chemotactic protein-2, γ-glutamyltransferase, Immunoglobulin G, Immunoglobulin A, Complement C3, Complement C2, Complement C1s, C-reactive protein, Alpha 1-microglobulin/bikunin precursor, Von Willebrandt Factor, Fibronectin, Vitronectin, Fibrinogen, Collagen I, Collagen III, Collagen IV, Collagen VII, Procollagen III, Laminin, Matrixmetalloproteinase 2, Apolipoprotein 4, Apolipoprotein A1, Ceruloplasmin, Thyreotyrein, Albumin, Alpha 2-macroglobulin precursor, Haptoglobin 1, α and β globin chains, Actin, and HSP 60, or functional variants thereof.

13. The method according to any of claims 10 to 12, wherein said sample from said mammal is serum and/or wound bed fluid, wherein said sample is collected before or after implantation of a silicone that is suitable for long-term implantations and coating of medical devices.

14. The method according to any of claims 10 to 13, further comprising a second sample as a control, wherein said control is either provided from the same mammal before or after implantation, or from a different mammal without any implantations of a medical silicone.

15. The method according to any of claims 10 to 14, wherein said method further comprises detecting the presence or amount of at least one nucleic acid having a nucleotide sequence encoding for a silicone-adhering polypeptide according to claim 6 or 7.

16. The method according to any of claims 10 to 15, wherein said method further comprises between step a) and step b) step a'), comprising contacting said sample with a silicone, and attaching said polypeptides to said silicone.

17. A diagnostic kit for detecting a response reaction of a mammal to a silicone, comprising materials, buffers, auxiliary agents, and substances for performing the method according to any of claims 10 to 16.

18. A method for monitoring a response reaction of a mammal to a silicone, comprising detecting the presence or a change in expression of a silicone-adhering polypeptide according to claim 6 or claim 7 using a method according to any of claims 10 to 16.

19. A method for screening substances that interfere with a silicone-adhering protein and/or polypeptide, comprising the steps of
a) providing a substance, wherein said substance is suspected to interfere with the adhering properties of a silicone-adhering polypeptide;
b) providing a silicone;
c) providing a silicone-adhering polypeptide; and;
d) analyzing the interfering properties of said substance through analyzing the adhesion of said silicone-adhering polypeptide to said silicone in the presence and absence of said substance.

20. The method according to claim 19, wherein said silicone-adhering polypeptide is selected of the group consisting of a polypeptide according to claim 6 or 7, Myeloid related protein 8, Myeloid related protein 14, HT018, PRO2619, a polypeptide identified according to any of claims 1 to 4, Isotype control, IgG-HRP, IgE-HRP, and IGA-HRP, AMBP protein precursor, Plasma-retinol binding protein, Kininogen, Eosinophil peroxidase, Fibroblast growth factor 11, TGF-β, Integrin-β4, T-cell receptor, NADH dehydrogenase, Coagulation factor V, α-spectrin, Monocyte chemotactic protein-2, γ-glutamyltransferase, Immunoglobulin G, Immunoglobulin A, Complement C3, Complement C2, Complement C1s, C reactive protein, Alpha 1-microglobulin/bikunin precursor, Von Willebrandt Factor, Fibronectin, Vitronectin, Fibrinogen, Collagen I, Collagen III, Collagen IV, Collagen VII, Procollagen III, Laminin, Matrixmetalloproteinase 2, Apolipoprotein 4, Apolipoprotein A1, Ceruloplasmin, Thyreotyrein, Albumin, Alpha 2-macroglobulin precursor, Haptoglobin 1, α and β globin chains, Actin, and HSP 60, or functional variants thereof.

21. A method for detecting cell adhesion to silicone, comprising
a) providing a sample of serum or wound bed fluid of a mammal;
b) providing of silicone; and
c) analyzing the amount or number of cells from said serum or wound bed fluid that are adhered to said silicone.


SEQUENCE LISTING

<110>  IDC Wick KG

<120>  Test Systems for the Analysis of Polypeptides and Cells adhering
       to Silicones

<130>  FB16436

<160>  4

<170>  PatentIn version 3.3

<210>  1
<211>  1032
<212>  PRT
<213>  Human

<400>  1

<210>  2
<211>  996
<212>  PRT
<213>  Human

<400>  2

Pro Gly Leu Leu Tyr Leu Ser Ser His Gly Leu Trp Leu Ile Thr Ile
1               5                   10                  15

Ala Lys Cys Gly Ile Leu Cys Ile Arg Asp Val Tyr Thr Leu Glu Thr
            20                  25                  30

Phe Ala Trp Cys Arg Ser His Ser His Gln Gly His Gly Ile Gln Ser
        35                  40                  45

Met Arg Ile Ser Met Asp Gly Gln Asn Ile Leu Val Asn Gly Arg Asp
    50                  55                  60

Asp Gly Thr Leu Val Tyr Leu Lys Trp Lys Arg Phe Gly Gly His Leu
65                  70                  75                  80

Ala Ser Glu Ile Leu Asp Tyr Tyr Gln Lys Leu Leu Ile Ser Leu Ser
                85                  90                  95

Ser Ala Met Asp Lys Glu Asn His Tyr Leu Ser Thr Thr Pro Lys Val
            100                 105                 110

Ser Val Asp Leu Gly Ser Asp Ser Glu His Thr Lys Gln Lys Ala Ser
        115                 120                 125

Thr Asp Leu Ser Gln Asp Glu Leu Val Leu Thr Asp Val Lys Lys Glu
    130                 135                 140

Ile Pro Trp Ile Gln Gln Lys Ser Gln Glu Ala Ile Lys Lys Glu Val
145                 150                 155                 160

Asn Leu Phe Ser Lys Lys Arg Lys Glu Ile Lys Gln Gly Ile Lys Ser
                165                 170                 175

Leu Ser Lys Thr Ile Leu Asn Met Met Glu Glu Asn Asp Lys Leu Glu
            180                 185                 190

Asn Ile Ala Lys Leu Asp Gln Gln Glu Phe Gly Leu Asp Leu Glu Glu
        195                 200                 205

Leu Glu Arg Leu His Asp Glu Ser Gln Glu Glu Val Ala Lys Met Ile
    210                 215                 220

Lys Asp Val Glu Met His Asn Leu Ala Lys Ser Tyr Leu Ala Glu Leu
225                 230                 235                 240

Ile Lys Glu Glu Cys Trp Asn Ser Met Ala Val Lys Gly Arg Ala Leu
                245                 250                 255

Lys Cys Phe His Ile Pro Cys Val Val Glu Asn Phe Pro Met Lys Ala
            260                 265                 270

Arg Thr Val Glu Glu Leu Lys Glu Leu Glu Arg Val Leu Gln Gln Lys
        275                 280                 285

Lys Ile Glu Ala Glu Cys Leu Lys Leu Arg Lys Glu Ile Val Glu Ala
    290                 295                 300

Gln Ser Gly Val Lys Leu Ile Lys Gln Arg His Glu Glu Asp Asp Glu
305                 310                 315                 320

Glu Glu Glu Glu Glu Asp Lys Thr Val Lys Tyr Ser Asn Leu Pro Asn
                325                 330                 335

Tyr Leu Leu Gly Ser Leu Ser Thr Asp Phe Gly Val Asp Thr Ser Leu
            340                 345                 350

Leu Ser Ser Gln Leu Glu Leu His Ser Arg Glu Glu Lys Ile Asn Gln
        355                 360                 365

Ile Ile Leu Leu Lys Asp Ile Ile Tyr Lys Val Lys Thr Val Phe Asn
    370                 375                 380

Asn Glu Phe Asp Ala Ala Tyr Lys Gln Lys Glu Phe Glu Ile Ala Arg
385                 390                 395                 400

Val Lys Glu Arg Asn Val Arg Ile Arg Glu Ile Ile Leu Asp Leu Glu
                405                 410                 415

Leu Glu Glu Ala Val Trp Gln Pro Glu Phe Glu Asp Cys Glu Lys Pro
            420                 425                 430

Glu Arg Thr Leu Val Val Gln Asp Glu Glu Ile Thr Ala His Lys His
        435                 440                 445

Ile Lys Pro Trp His Lys Ala Lys Glu Leu Ile Val Asn His Glu Lys
    450                 455                 460

Glu His Trp Leu Leu Ile Gln Asp Ala Ser Thr Arg Leu Arg Ala Leu
465                 470                 475                 480

Met Asp Met Met Gly Gly Val Leu Glu Val Lys Lys Glu Asp Ile Leu
                485                 490                 495

Arg Met Val Ile Pro Gln Pro Ala Phe Met Ala Lys Pro Asp Ala Val
            500                 505                 510

Trp Thr Glu Glu Glu Arg Lys Gln Phe Lys Asp Tyr Glu Lys Lys Val
        515                 520                 525

Lys Glu Leu Asn Glu Glu Arg Asp Lys Tyr Arg Lys Ser Leu Glu Ala
    530                 535                 540

Glu Leu Lys Lys Leu Gln Asn Ser Ile Gln Glu Ser Thr Gln Ala Phe
545                 550                 555                 560

Asp Glu His Leu Lys Arg Leu Phe Glu Arg Arg Val Lys Ala Glu Met
                565                 570                 575

Val Thr Asn Gln Glu Glu Leu Lys Ile Ser Asn Leu Ala Phe Ser Leu
            580                 585                 590

Leu Leu Asp Glu Glu Leu Ser Ser Arg Glu Lys Phe Leu Asn Asn Tyr
        595                 600                 605

Leu Thr Arg Lys Gln His Glu Lys Val Met Asp Arg Ser Phe Lys Lys
    610                 615                 620

Glu Phe Ser Glu Ile Pro Gly His Gln Val Asp Ile Leu Tyr Lys Leu
625                 630                 635                 640

Phe Lys Arg Arg Pro Arg Ile Ser Lys Gln Lys Thr His Ser Glu Thr
                645                 650                 655

Thr Ser Val Val Pro Phe Gly Glu Leu Pro Gly Ser Gly Lys Leu Asn
            660                 665                 670

Lys Asp Ala Phe Ala Gln Leu Met Lys Ala Met Asp Glu Leu Asp Asn
        675                 680                 685

Ile Ser Asn Met Pro Glu Gly Leu Asp Pro Leu Val Trp Asn His Phe
    690                 695                 700

Cys Met Thr Arg Arg Ala Lys Val Glu Asn Glu Gln Lys Val Lys Gln
705                 710                 715                 720

Lys Ala Ala Asp Leu Leu Glu Met Ala Thr Phe Leu Gln Lys Arg Val
                725                 730                 735

Glu Glu Glu Glu Lys Val Gln Gln Glu Ile Glu Arg Val Phe His Glu
            740                 745                 750

Leu Ile Leu Leu Gln Glu Glu Lys Val Arg Phe Gln Leu Asn Leu Thr
        755                 760                 765

Ile Gln Ile Leu Leu Lys Gln Gly Gln Val Glu Leu Glu Asn Phe Gln
    770                 775                 780

Leu Val Leu Glu Tyr Ser Asp Ala Ile Leu Ile Asn Lys Asn Ile Ile
785                 790                 795                 800

Glu Asp Leu Asn Ser Val Ile Arg Thr Gln Gly Gln Lys Lys Val Ala
                805                 810                 815

Ser Met Met Glu Ser Lys Asp Val His Lys Arg Ile Leu Gln Ile Glu
            820                 825                 830

Trp Glu His Lys Lys Met Glu Met Glu Arg Glu Asp Leu Asn Gln Lys
        835                 840                 845

Ala Trp Asp Ile Gln Met Leu Phe Phe Ser Arg Asp Arg Gln Lys Tyr
    850                 855                 860

Leu Asn Glu Pro Asn Tyr Glu Ala Leu Ile Ser Ile Gln Ile Gly Ile

865                 870                 875                 880

Met Glu Gln Thr Ile Ala Val Leu Asp Lys Met His Lys Lys Asn Val
                885                 890                 895

Glu Asn Cys Lys Lys Leu Leu Lys Lys Leu Gly Lys Phe Ser Asn Gln
            900                 905                 910

Lys Asp Ile Ala Asn Tyr Ala Leu Ser Cys Asn Leu Arg Glu Glu Leu
        915                 920                 925

Val Ala Val Ser Glu Arg Lys Asp Ile Cys Asn Ala Met Gly Ser Lys
    930                 935                 940

Leu Thr Cys Glu Lys Ile Val Lys Glu Arg Tyr Glu Asn Met Met Gln
945                 950                 955                 960

Gln Gln Lys Leu Thr Asn Ile Ser Lys Gln Gln Ala Glu Gln Ile Ser
                965                 970                 975

Ile Leu Gln Thr Glu Val Glu Arg Leu Arg Met Lys Thr Phe Pro Ala
            980                 985                 990

Leu Val Gln Met
        995

<210>  3
<211>  347
<212>  PRT
<213>  Human

<400>  3

Met Val Ala Ser Thr Val Ala Asn Gly Pro Arg Ser Pro Trp Asp Ala
1               5                   10                  15

Ile Ser Leu Ile Ile Met Leu Arg Ile Trp Arg Val Lys Arg Val Ile
            20                  25                  30

Asp Ala Tyr Val Leu Pro Val Lys Leu Glu Met Glu Met Val Ile Gln
        35                  40                  45

Gln Tyr Glu Lys Ala Lys Val Ile Gln Asp Glu Gln Leu Glu Arg Leu
    50                  55                  60

Thr Gln Ile Cys Gln Glu Gln Gly Phe Glu Ile Arg Gln Leu Arg Ala
65                  70                  75                  80

His Leu Ala Gln Gln Asp Leu Asp Leu Ala Ala Glu Arg Glu Ala Ala

85                  90                  95

Leu Gln Ala Pro His Val Leu Ser Gln Pro Arg Ser Arg Phe Lys Val
            100                 105                 110

Leu Glu Ala Gly Thr Trp Asp Glu Glu Thr Ala Ala Glu Ser Val Val
        115                 120                 125

Glu Glu Leu Gln Pro Ser Gln Glu Ala Thr Met Lys Asp Asp Met Asn
    130                 135                 140

Ser Tyr Ile Ser Gln Tyr Tyr Asn Gly Pro Ser Ser Asp Ser Gly Val
145                 150                 155                 160

Pro Glu Pro Ala Val Cys Met Val Thr Thr Ala Ala Ile Asp Ile His
                165                 170                 175

Gln Pro Asn Ile Ser Ser Asp Leu Phe Ser Leu Asp Met Pro Leu Lys
            180                 185                 190

Leu Gly Gly Asn Gly Thr Ser Ala Thr Ser Glu Ser Ala Ser Arg Ser
        195                 200                 205

Ser Val Thr Arg Ala Gln Ser Asp Ser Ser Gln Thr Leu Gly Ser Ser
    210                 215                 220

Met Asp Cys Ser Thr Ala Arg Glu Glu Pro Ser Ser Glu Pro Gly Pro
225                 230                 235                 240

Ser Pro Pro Pro Leu Pro Ser Gln Gln Gln Val Glu Glu Ala Thr Val
                245                 250                 255

Gln Asp Leu Leu Ser Ser Leu Ser Glu Asp Pro Cys Pro Ser Gln Lys
            260                 265                 270

Ala Leu Asp Pro Ala Pro Leu Ala Arg Pro Ser Pro Ala Gly Ser Ala
        275                 280                 285

Gln Thr Ser Pro Glu Leu Glu His Arg Val Ser Leu Phe Asn Gln Lys
    290                 295                 300

Asn Gln Glu Gly Phe Thr Val Phe Gln Ile Arg Pro Val Ile His Phe
305                 310                 315                 320

Gln Pro Thr Val Pro Met Leu Glu Asp Lys Phe Arg Ser Leu Glu Ser
                325                 330                 335

Lys Glu Gln Lys Leu His Arg Val Pro Glu Ala
            340                 345

<210> 4
<211> 232
<212> PRT
<213> Human

<400> 4

Met Gly Ser Val Leu Ser Thr Asp Ser Gly Lys Ser Ala Pro Ala Ser
1               5                   10                  15

Ala Thr Ala Arg Ala Leu Glu Arg Arg Arg Asp Pro Glu Leu Pro Val
            20                  25                  30

Thr Ser Phe Asp Cys Ala Val Cys Leu Glu Val Leu His Gln Pro Val
        35                  40                  45

Arg Thr Arg Cys Gly His Val Phe Cys Arg Ser Cys Ile Ala Thr Ser
    50                  55                  60

Leu Lys Asn Asn Lys Trp Thr Cys Pro Tyr Cys Arg Ala Tyr Leu Pro
65                  70                  75                  80

Ser Glu Gly Val Pro Ala Thr Asp Val Ala Lys Arg Met Lys Ser Glu
                85                  90                  95

Tyr Lys Asn Cys Ala Glu Cys Asp Thr Leu Val Cys Leu Ser Glu Met
            100                 105                 110

Arg Ala His Ile Arg Thr Cys Gln Lys Tyr Ile Asp Lys Tyr Gly Pro
        115                 120                 125

Leu Gln Glu Leu Glu Glu Thr Ala Ala Arg Cys Val Cys Pro Phe Cys
    130                 135                 140

Gln Arg Glu Leu Tyr Glu Asp Ser Leu Leu Asp His Cys Ile Thr His
145                 150                 155                 160

His Arg Ser Glu Arg Arg Pro Val Phe Cys Pro Leu Cys Arg Leu Ile
                165                 170                 175

Pro Asp Glu Asn Pro Ser Ser Phe Ser Gly Asn Leu Ile Arg His Leu
            180                 185                 190

Gln Val Ser His Thr Leu Phe Tyr Asp Asp Phe Ile Asp Phe Asn Ile
        195                 200                 205

Ile Glu Glu Ala Leu Ile Arg Arg Val Leu Asp Arg Ser Leu Leu Glu
    210                 215                 220

Tyr Val Asn His Ser Asn Thr Thr
225                 230